(19) Europäisches Patentamt
European Patent Office
Office européen des brevets

(11) EP 2 753 676 B1

(12) # EUROPEAN PATENT SPECIFICATION

(45) Date of publication and mention of the grant of the patent:
**23.01.2019 Bulletin 2019/04**

(21) Application number: **12746283.6**

(22) Date of filing: **07.08.2012**

(51) Int Cl.:
***C07C 13/28*** *(2006.01)* ***C07C 15/50*** *(2006.01)*
***C07C 25/22*** *(2006.01)* ***C09K 19/20*** *(2006.01)*
***C09K 19/30*** *(2006.01)* ***C09K 19/32*** *(2006.01)*
***C07C 15/54*** *(2006.01)*

(86) International application number:
**PCT/EP2012/003363**

(87) International publication number:
**WO 2013/034227 (14.03.2013 Gazette 2013/11)**

(54) **LIQUID-CRYSTALLINE MEDIUM AND HIGH-FREQUENCY COMPONENTS COMPRISING SAME**
FLÜSSIGKRISTALLINES MEDIUM UND HOCHFREQUENZBAUTEILE DAMIT
MILIEU CRISTAL LIQUIDE ET COMPOSANTS HAUTES FRÉQUENCES LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.09.2011 EP 11007161**

(43) Date of publication of application:
**16.07.2014 Bulletin 2014/29**

(73) Proprietor: **Merck Patent GmbH**
**64293 Darmstadt (DE)**

(72) Inventors:
- **MANABE, Atsutaka**
  **64625 Bensheim (DE)**
- **KLASS, Dagmar**
  **64291 Darmstadt (DE)**

(56) References cited:
**EP-A2- 1 479 748**
**EP-A2- 2 399 972**
**WO-A1-01/12751**
**CN-A- 102 010 718**
**DE-A1- 10 229 829**
**DE-A1-102004 002 418**
**DE-A1-102010 035 987**
**DE-A1-102010 047 404**
**DE-A1-102010 047 702**
**JP-A- H06 298 686**
**JP-A- 2005 232 455**
**JP-A- 2007 051 275**

- **WU S-T ET AL: "POLARIZED UV SPECTROSCOPY OF CONJUGATED LIQUID CRYSTALS", JOURNAL OF APPLIED PHYSICS, AMERICAN INSTITUTE OF PHYSICS. NEW YORK, US, vol. 68, no. 1, 1 July 1990 (1990-07-01), pages 78-85, XP001042050, ISSN: 0021-8979, DOI: 10.1063/1.347073**
- **CHRISTIAN WERNER ET AL: "1,x-Elimination Reactions: Extending the Limits of a Classical Organic Reaction", CHEMISTRY - A EUROPEAN JOURNAL, vol. 13, no. 34, 26 November 2007 (2007-11-26), pages 9462-9477, XP55042319, ISSN: 0947-6539, DOI: 10.1002/chem.200700827**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to liquid-crystalline media and to high-frequency components comprising same, especially microwave components for high-frequency devices, such as devices for shifting the phase of microwaves, in particular for microwave phased-array antennas.

Liquid-crystalline media have been used for some time in electro-optical displays (liquid crystal displays - LCDs) in order to display information.

Recently, however, liquid-crystalline media have also been proposed for use in components for microwave technology, such as, for example, in DE 10 2010 047404 A1, DE 10 2010 047702 A1, DE 10 2010 035987 A1, WO 2011/009524 A8, DE 10 2004 029 429 A and in JP 2005-120208 (A).

As a typical microwave application, the concept of the inverted microstrip line as described by K.C. Gupta, R. Garg, I. Bahl and P. Bhartia: Microstrip Lines and Slotlines, 2nd ed., Artech House, Boston, 1996, is employed, for example, in D. Dolfi, M. Labeyrie, P. Joffre and J.P. Huignard: Liquid Crystal Microwave Phase Shifter. Electronics Letters, Vol. 29, No. 10, pp. 926-928, May 1993, N. Martin, N. Tentillier, P. Laurent, B. Splingart, F. Huert, PH. Gelin, C. Legrand: Electrically Microwave Tuneable Components Using Liquid Crystals. 32nd European Microwave Conference, pp. 393-396, Milan 2002, or in Weil, C.: Passiv steuerbare Mikrowellenphasenschieber auf der Basis nichtlinearer Dielektrika [Passively Controllable Microwave Phase Shifters based on Nonlinear Dielectrics], Darmstädter Dissertationen D17, 2002, C. Weil, G. Lüssem, and R. Jakoby: Tuneable Invert-Microstrip Phase Shifter Device Using Nematic Liquid Crystals, IEEE MTT-S Int. Microw. Symp., Seattle, Washington, June 2002, pp. 367-370, together with the commercial liquid crystal K15 from Merck KGaA. C. Weil, G. Lüssem, and R. Jakoby: Tuneable Invert-Microstrip Phase Shifter Device Using Nematic Liquid Crystals, IEEE MTT-S Int. Microw. Symp., Seattle, Washington, June 2002, pp. 367-370, achieve phase shifter qualities of 12°/dB at 10 GHz with a control voltage of about 40 V therewith. The insertion losses of the LC, i.e. the losses caused only by the polarisation losses in the liquid crystal, are given as approximately 1 to 2 dB at 10 GHz in Weil, C.: Passiv steuerbare Mikrowellenphasenschieber auf der Basis nichtlinearer Dielektrika [Passively Controllable Microwave Phase Shifters based on Nonlinear Dielectrics], Darmstädter Dissertationen D17, 2002. In addition, it has been determined that the phase shifter losses are determined primarily by the dielectric LC losses and the losses at the waveguide junctions. T. Kuki, H. Fujikake, H. Kamoda and T. Nomoto: Microwave Variable Delay Line Using a Membrane Impregnated with Liquid Crystal. IEEE MTT-S Int. Microwave Symp. Dig. 2002, pp. 363-366, June 2002, and T. Kuki, H. Fujikake, T. Nomoto: Microwave Variable Delay Line Using Dual-Frequency Switching-Mode Liquid Crystal. IEEE Trans. Microwave Theory Tech., Vol. 50, No. 11, pp. 2604-2609, November 2002, also address the use of polymerised LC films and dual-frequency switching-mode liquid crystals in combination with planar phase shifter arrangements.

A. Penirschke, S. Müller, P. Scheele, C. Weil, M. Wittek, C. Hock and R. Jakoby: "Cavity Perturbation Method for Characterization of Liquid Crystals up to 35GHz", 34th European Microwave Conference - Amsterdam, pp. 545-548 describe, inter alia, the properties of the known single liquid crystal substance K15 (Merck KGaA, Germany) at a frequency of 9 GHz.

A. Gaebler, F. Goelden, S. Müller, A. Penirschke and R. Jakoby "Direct Simulation of Material Permittivites using an Eigen-Susceptibility Formulation of the Vector Variational Approach", 12MTC 2009 - International Instrumentation and Measurement Technology Conference, Singapore, 2009 (IEEE), pp. 463-467, describe the corresponding properties of the known liquid-crystalline mixture E7 (likewise Merck KGaA, Germany).

DE 10 2004 029 429 A describes the use of liquid-crystalline media in microwave technology, inter alia in phase shifters. DE 10 2004 029 429 A has already investigated liquid-crystalline media with respect to their properties in the corresponding frequency range.

Liquid-crystalline media comprising for example compounds of the formula below,

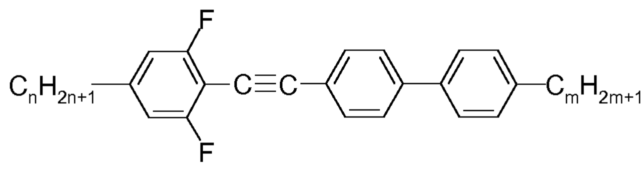

are used as a host mixture for the investigation of compounds, which are suggested for the usage in components for microwave applications and are described in F. Golden, "Liquid Crystal Based Microwave Components with Fast Response Times: Materials, Technology, Power Handling Capability", Dissertation, Technische Universität Darmstadt, 2009, (**D17**), A. Lapanik, "Single compounds and mixtures for microwave applications, Dielectric, microwave studies on selected systems", Dissertation, Technische Universität Darmstadt, 2009, (**D17**),
"Nematic LC mixtures with high birefringence in microwave region",

A. Lapanik, F. Golden, S. Müller, A. Penirschke, R. Jakoby und W. Haase, Frequenz 2011, 65, 15-19, "Highly birefringent nematic mixtures at room temperature for microwave applications", A. Lapanik, F. Golden, S. Müller, R. Jakoby und W. Haase, Journal of Optical Engineering, published online, as well as in the laid-open documents DE 10 2010 045 370.6 und DE 10 2010 051 508.0.

However, these compositions are afflicted with serious disadvantages. Most of them result, besides other deficiencies, in slow switching performances.

Fast switching performances are especially needed for mobile applications e.g. for receiving and sending communication, television or video to vehicles like ships, planes, trains and cars. Other preferred applications are short-distance antennas of the wireless routers to the laptop PCs, tablet computers and mobile devices.

For these applications, liquid-crystalline media having particular, hitherto rather unusual, uncommon properties, or combinations of properties, are required.

Novel liquid-crystalline media having improved properties are thus necessary. In particular, fast switching liquid-crystalline media with low values for the rotational viscosity ($\gamma_1$) are required, which also exhibit an acceptable tunability ($\tau$).

In this context, the dielectric anisotropy in the microwave region is defined as

$$\Delta\varepsilon_r \equiv (\varepsilon_{r,||} - \varepsilon_{r,\perp}).$$

The tuneability ($\tau$) is defined as

$$\tau \equiv (\Delta\varepsilon_r / \varepsilon_{r,||}).$$

The material quality ($\eta$) is defined as

$$\eta \equiv (\tau / \tan \delta_{\varepsilon\ r,max.}),$$

where the maximum dielectric loss is

$$\tan \delta_{\varepsilon\ r,max.} \equiv \max. \{ \tan \delta_{\varepsilon\ r,\perp} ; \tan \delta_{\varepsilon\ r,||} \}.$$

In addition, there is a demand for an improvement in the low-temperature behaviour of the components. Both an improvement in the operating properties and in the shelf life is necessary here.

Therefore, there is a considerable demand for liquid-crystalline media having suitable properties for corresponding practical applications.

Surprisingly, it has now been found that it is possible to achieve liquid-crystalline medium having a suitably high $\Delta\varepsilon$, broad nematic phase ranges, a suitable high $\Delta n$ and especially, low rotational rotation viscosity in connection with an acceptable tunability that do not have the disadvantages of the prior-art materials, or at least only do so to a considerably reduced extent.

These improved liquid-crystalline media in accordance with the present invention, comprise at least one compound selected from the group of compounds of formulae IB-1, IB-2 and IB-3, as in Claim 1 and defined below, and
one or more compounds of formula II

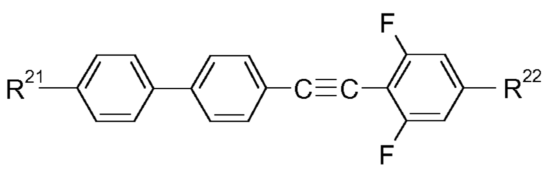

II

wherein
$R^{21}$ and $R^{22}$ have one of the meanings indicated for $R^{11}$, and one or more compounds of formula III

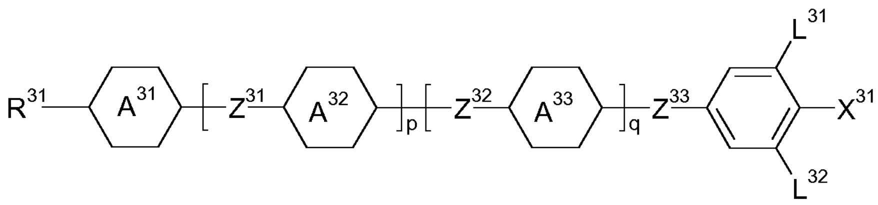 III

wherein

$R^{31}$ has one of the meanings indicated for $R^{11}$,

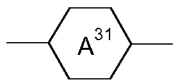 to 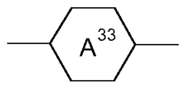

denote independently of one another, and in case $A^{32}$ and/or $A^{33}$ is/are present twice, also these independently of one another denote

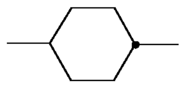, 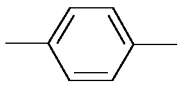, 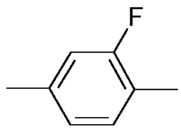,

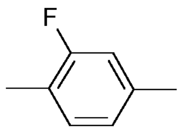, 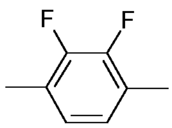 or 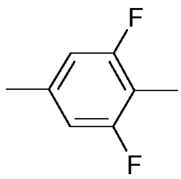,

$L^{31}$ and $L^{32}$ denote independently of one another H or F,

$X^{31}$ denotes halogen, halogenated alkyl or alkoxy with 1 to 3 C-atoms or halogenated alkenyl or alkenyloxy with 2 or 3 C-atoms or CN or NCS,

$Z^{31}$ to $Z^{32}$ denote independently of one another, and in case $Z^{31}$ and/or $Z^{32}$ is/are present twice, also these independently of one another denote $-CH_2CH_2-$, $-CF_2CF_2-$, -COO-, *trans*- -CH=CH-, *trans*- -CF=CF-, $-CH_2O-$, $-CF_2O-$ or a single bond,

$Z^{33}$ denotes $-CF_2O-$, and

p and q denote independently of one another 0, 1, 2 or 3, and wherein $p + q \geq 1$.

The invention further relates to a component for high-frequency technology comprising a liquid-crystalline medium as described above and below. In this context, both high-frequency technology and hyper-frequency technology denote applications having frequencies in the range from 1 MHz to 1 THz, preferably from 1 GHz to 500 GHz, more preferably 2 GHz to 300 GHz, comprising a component for high-frequency technology comprising a liquid-crystalline medium as described above and below, suitable for operation in the microwave range.

The invention further relates to the use of liquid-crystalline media as described above and below in a component for high-frequency technology.

The invention further relates to a microwave device, comprising a component as described above and below.

Said devices and components include, without limitation, phase shifters, varactors, wireless and radio wave antenna arrays, phased array antennas, reflectarray antenna, adaptable matching circuit, tunable filter and others.

In particular, the liquid-crystalline media of this invention show the following advantageous properties:

- they exhibit a high birefringence Δn, usually an Δn 0.230 or more, and/or
- they exhibit a acceptable nematic phase ranges usually up to 80°C or more, and/or
- they exhibit high dielectric anisotropy values in the microwave range (19 GHz), usually 0.50 or more, and/or
- they exhibit high values for the material quality (η) of 7 or more, and/or
- they exhibit low values for the rotational viscosity usually below 600 mPa·s or less together with acceptable values for tunability of 0.15 or more.

The media are especially suitable for mass production and can be processed using industry standard equipment.
Furthermore, exhibit the liquid-crystalline media according to the present invention good storage stability and good low temperature stability.
In a preferred embodiment, the medium comprises one, two or more compounds of the formulae IA or IC,

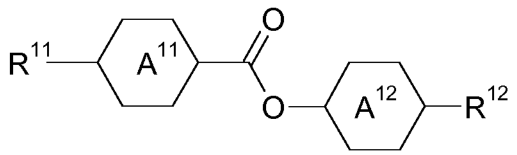

IA

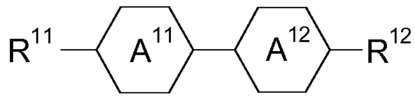

IC

wherein $R^{11}$ and $R^{12}$ have the meanings indicated above in formula IB,

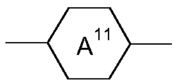

and

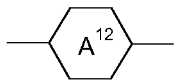

have the meanings indicated above in formula IB,
**[0028]** The compounds of the formula IA are preferably selected from the group of compounds of formulae IA-1 to IA-6,

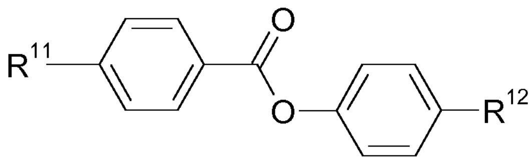

IA-1

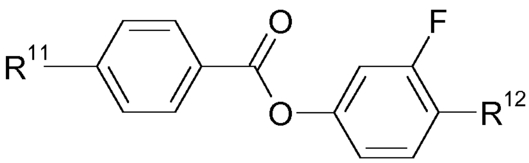

IA-2

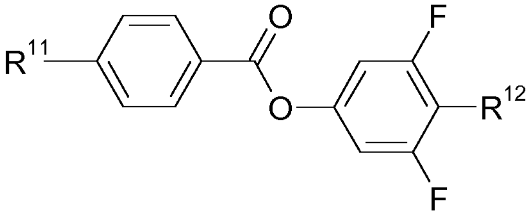

IA-3

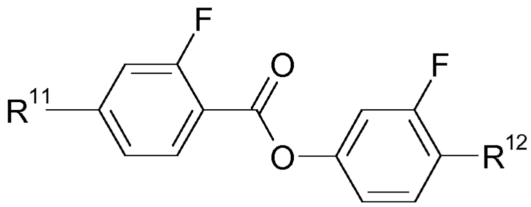

IA-4

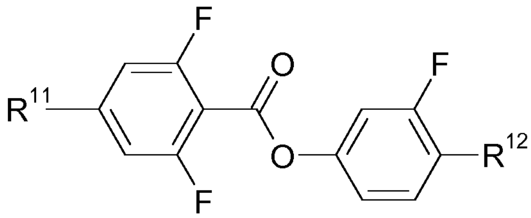

IA-5

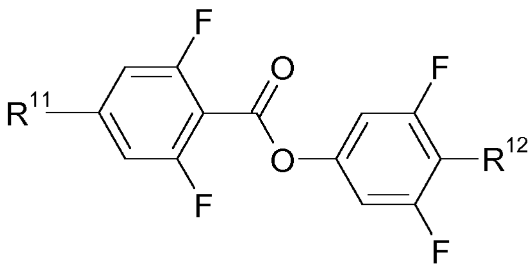

IA-6

wherein

$R^{11}$ has the meaning indicated above in formula IB and preferably denotes $C_nH_{2n+1}$ or $O-C_nH_{2n+1}$ or $CH_2=CH-(CH_2)_Z$, and wherein

n denotes an integer in the range from 1 to 7 and preferably from 1 to 5, and

z denotes 0, 1, 2, 3 or 4, preferably 0 or 2, and

$R^{12}$ has the meaning indicated above in formula IB and preferably denotes -F, -Cl, -CN or -NCS, more preferably -CN.

More preferred compounds of formulae IA-1 to IA-6 are selected from compounds of formulae IA-1 and/or IA-2 and/or IA-3, even more preferred are compounds of formula IA-2.

The media in accordance with the present invention comprise at least one compound selected from the group of compounds of formulae IB-1, IB-2 and IB-3,

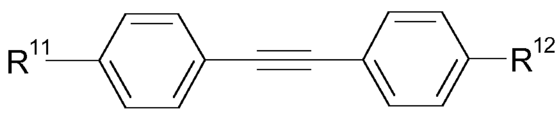

IB-1

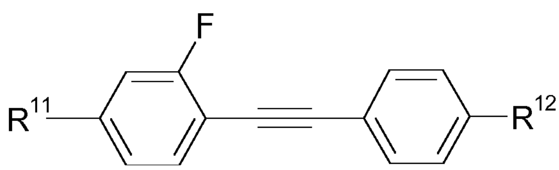

IB-2

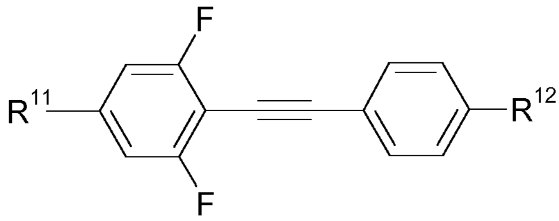

IB-3

wherein

$R^{11}$ $C_nH_{2n+1}$,

$R^{12}$ $C_mH_{2m+1}$ and wherein

m and n independently of one another, denote an integer in the range from 1 to 7 and preferably from 1 to 5, and

Most preferred compounds of formula IB are compounds of formula IB-1.

Compounds of the formula IC are preferably selected from the group of the compounds of formulae IC-1 to IC-3, most preferably of formula IC-1

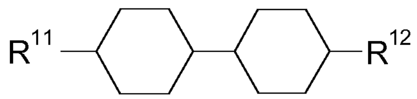

IC-1

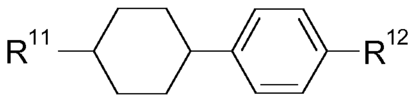

IC-2

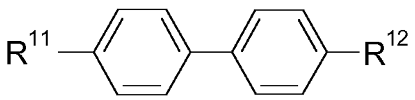

IC-3

wherein

$R^{11}$ has the meaning indicated above in formula IB and preferably denotes $C_nH_{2n+1}$ or $O\text{-}C_nH_{2n+1}$ or $CH_2\text{=}CH\text{-}(CH_2)_Z$ or $(CH_2)_Z\text{-}C{\equiv}C\text{-}$ $C_nH_{2n+1}$ and

$R^{12}$ has the meaning indicated above in formula IB and preferably denotes $C_mH_{2m+1}$ or $O\text{-}C_mH_{2m+1}$ or $(CH_2)_Z\text{-}CH\text{=}CH_2$, $(CH_2)_Z\text{-}C{\equiv}C\text{-}$ $C_mH_{2m+1}$ and wherein

n and m independently of one another, denote an integer in the range from 1 to 7 and preferably from 1 to 5, and

z denotes 0, 1, 2, 3 or 4, preferably 0 or 2.

Preferred combinations of ($R^{11}$ and $R^{12}$), in particular in formulae IC-1 and IC-3, are ($C_nH_{2n+1}$ and $C_mH_{2m+1}$), ($C_nH_{2n+1}$ and $O\text{-}C_mH_{2m+1}$), ($CH_2\text{=}CH\text{-}(CH_2)_Z$ and $C_mH_{2m+1}$), ($CH_2\text{=}CH\text{-}(CH_2)_Z$ and $O\text{-}C_mH_{2m+1}$), ($C_nH_{2n+1}$ and $(CH_2)_Z\text{-}CH\text{=}CH_2$), (($C_nH_{n+1}$)-$CH\text{=}CH\text{-}(CH_2)_Z$ and $O\text{-}C_mH_{2m+1}$) and (($C_nH_{2n+1}$ and $(CH_2)_Z\text{-}CH\text{=}CH\text{-}(C_mH_{2m+1})$)).

In another preferred embodiment, the compounds of the formula IC are selected from the group of compounds of the formulae IC-4 to IC-12:

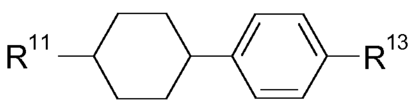

IC-4

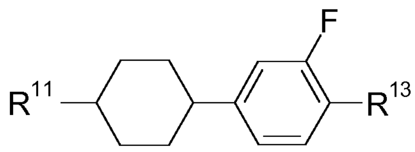

IC-5

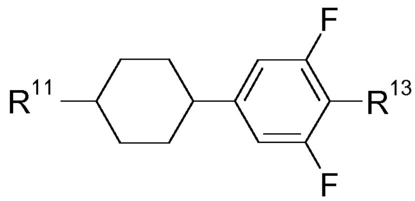

IC-6

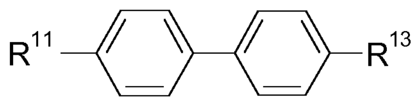

IC-7

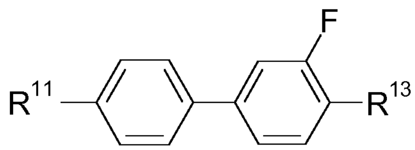

IC-8

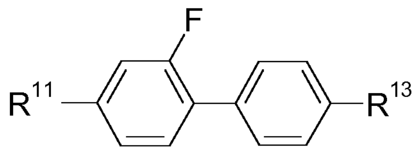

IC-9

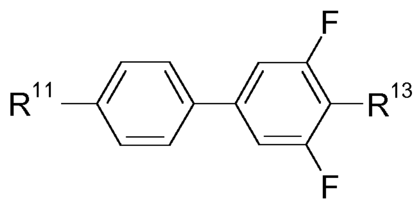

IC-10

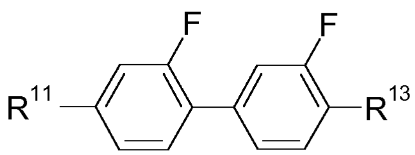

IC-11

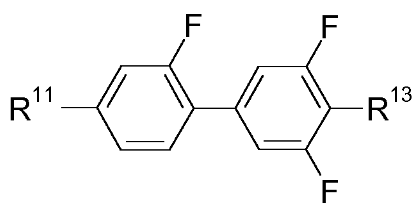

IC-12

wherein

$R^{11}$ has the meaning indicated above in formula IB and preferably denotes $C_nH_{2n+1}$ or O-$C_nH_{2n+1}$ or $CH_2$=CH-$(CH_2)_Z$ or $(CH_2)_Z$-C≡C- $C_nH_{2n+1}$ and

n denotes an integer in the range from 1 to 7 and preferably from 1 to 5, and

z denotes 0, 1, 2, 3 or 4, preferably 0 or 2, and

$R^{13}$ denotes -F, -Cl, -CN or -NCS, preferably -CN or -NCS.

Especially preferred compounds of formulae IC-4 to IC-13 are IC-4, IC-5, IC-6, IC-7, IC-9, IC-10 and IC-11.

Compounds of the formula II are preferably selected from the group of compounds of formulae IIA to IIE,

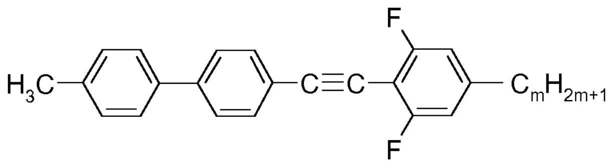

IIA

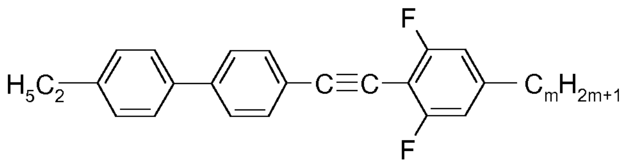

IIB

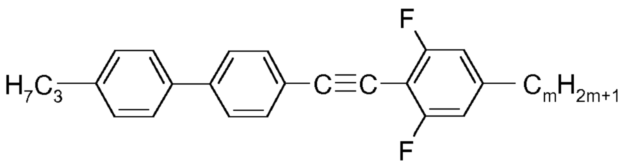

IIC

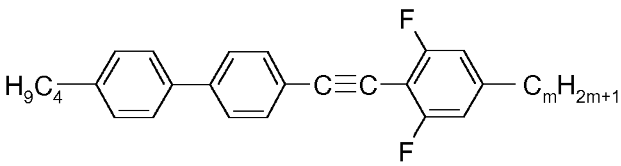

IID

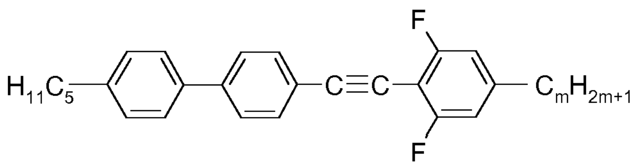

IIE

m denotes an integer in the range from 1 to 7 and preferably from 1 to 5.

Especially preferred compounds of formulae IIA to IIE are compounds of formulae IIC and IID, and wherein m denotes 2 and 4.

Compounds of the formula III are preferably selected from the group of compounds of formulae IIIA to IIIC,

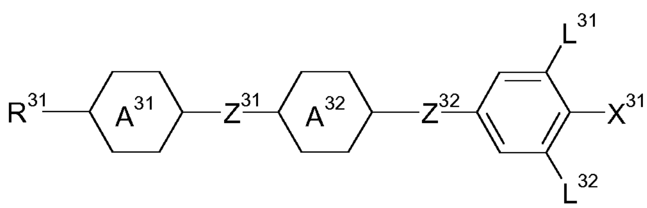

IIIA

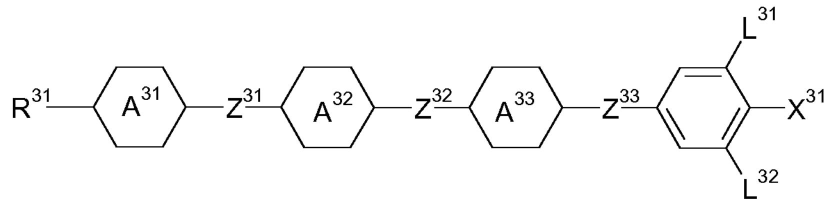

IIIB

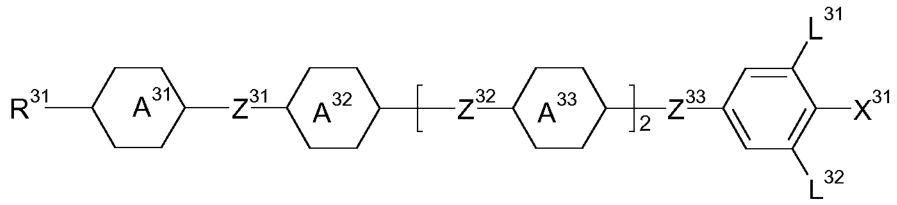

IIIC

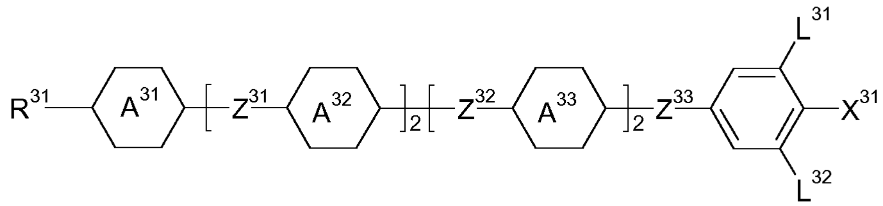

IIID

wherein $R^{31}$, $A^{31}$ to $A^{33}$, $Z^{31}$ to $Z^{33}$, $L^{31}$ and $L^{32}$ and $X^{31}$ have the meanings indicated above in formula III.

Preferred compounds of formula IIIA are selected from compounds of formulae IIIA-14 to IIIA-31 as indicated below:

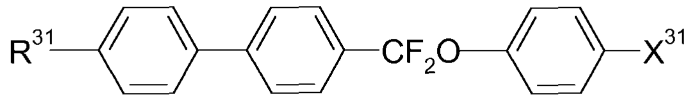

IIIA-14

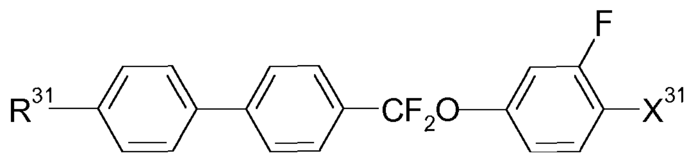

IIIA-15

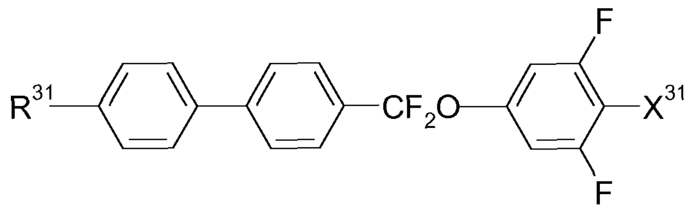

IIIA-16

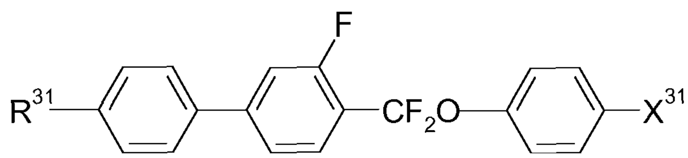

IIIA-17

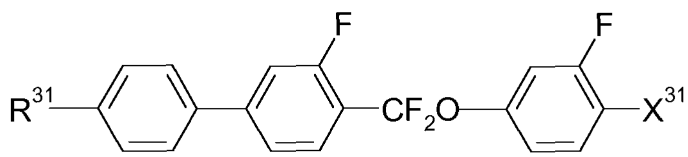

IIIA-18

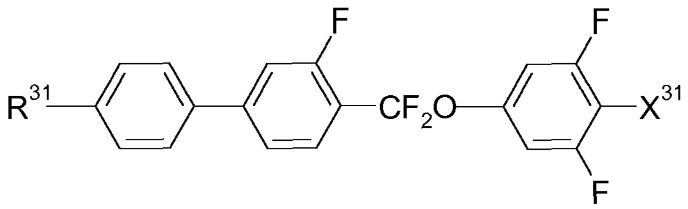

IIIA-19

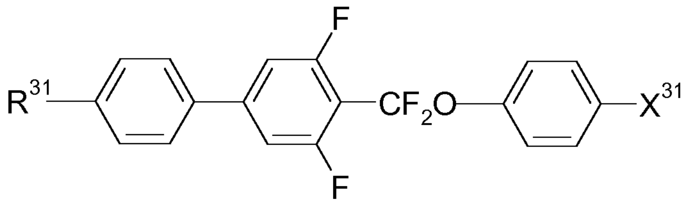

IIIA-20

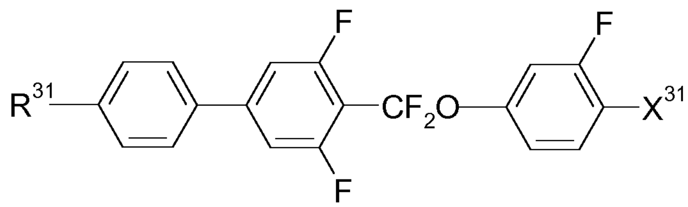

IIIA-21

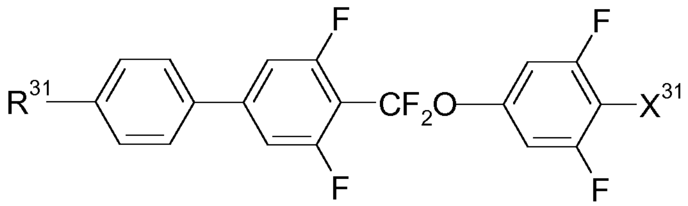

IIIA-22

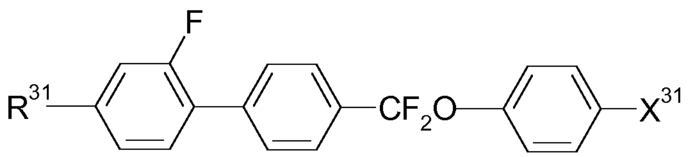

IIIA-23

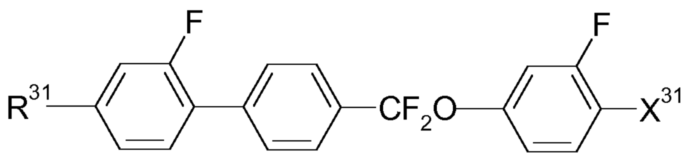

IIIA-24

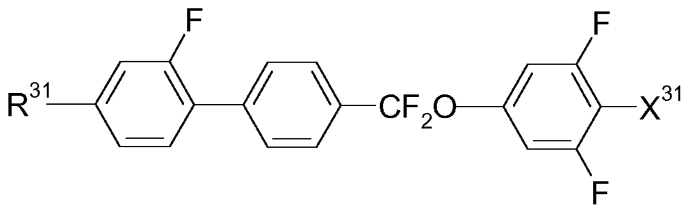

IIIA-25

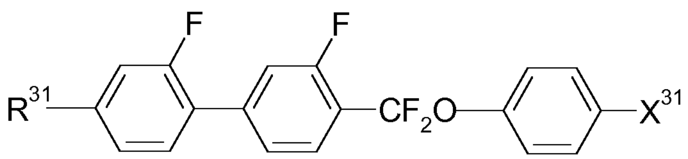

IIIA-26

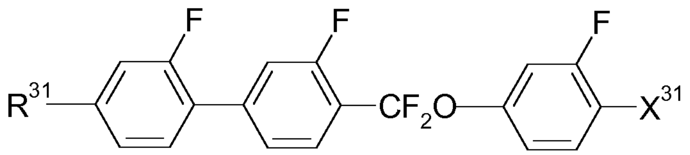

IIIA-27

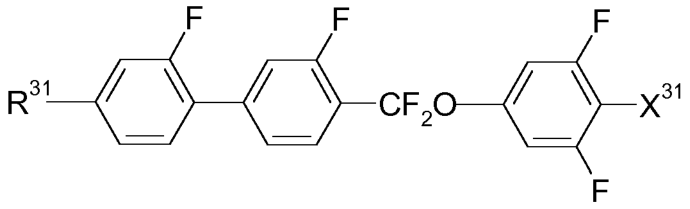

IIIA-28

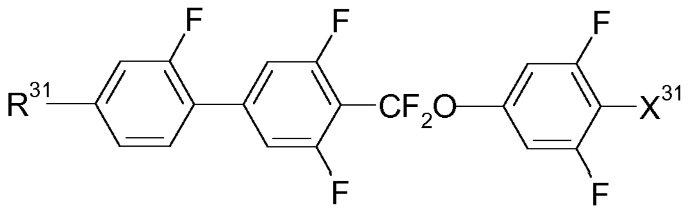

IIIA-29

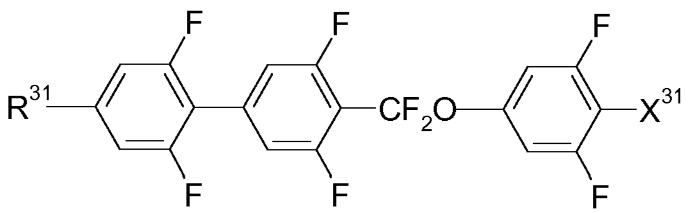

IIIA-30

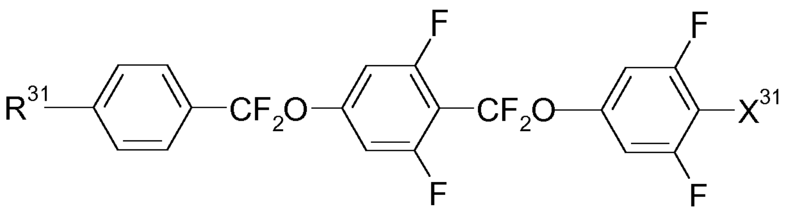

IIIA-31

wherein $R^{31}$ and $X^{31}$ have the meanings indicated above in formula III.

Especially preferred compounds of formulae IIIA-14 to IIIA-31 are compounds of formula IIIA-22.

Compounds of formula IIIB are preferably selected from the group of compounds of formulae IIIB-6 to IIIB-11 as indicated below:

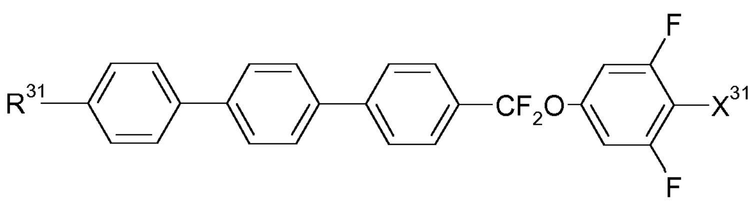

IIIB-6

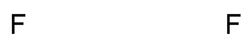

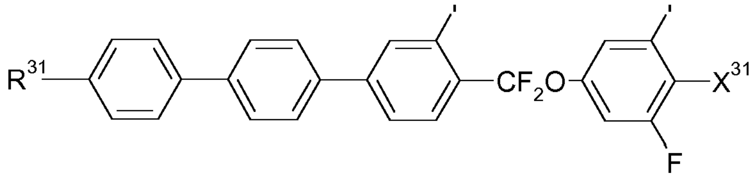

IIIB-7

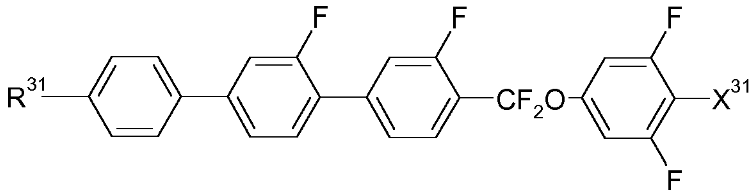

IIIB-8

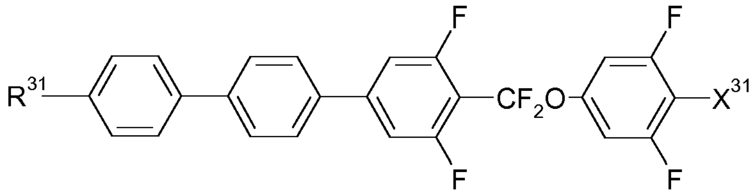

IIIB-9

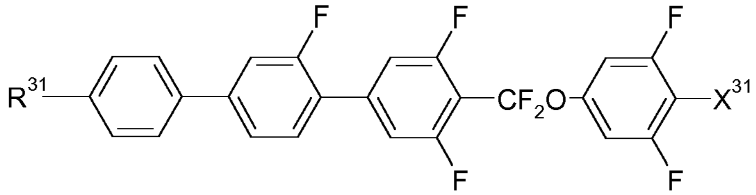

IIIB-10

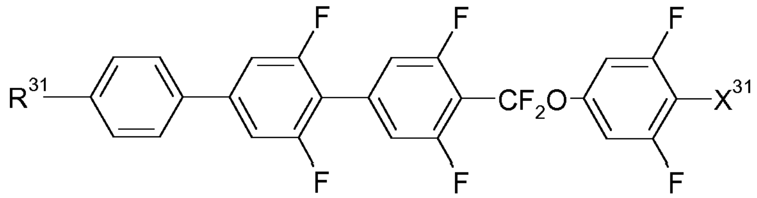

IIIB-11

wherein $R^{31}$ and $X^{31}$ have the meanings indicated above in formula III.

Especially preferred compounds of formulae IIIB-6 to IIIB-11 are compounds of formula IIIB-10. Preferred compounds of formula IIIC are selected of formulae IIIC-1 to IIIC-9 as indicated below:

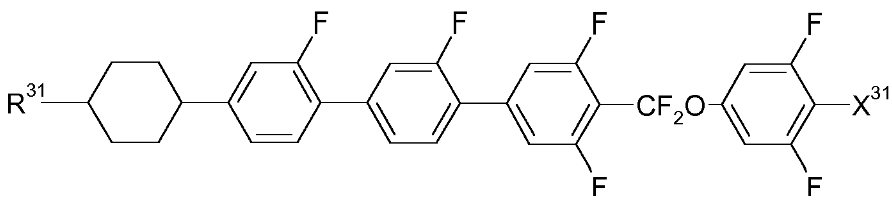

IIIC-1

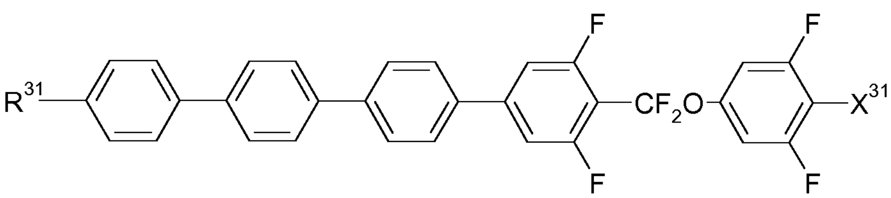

IIIC-2

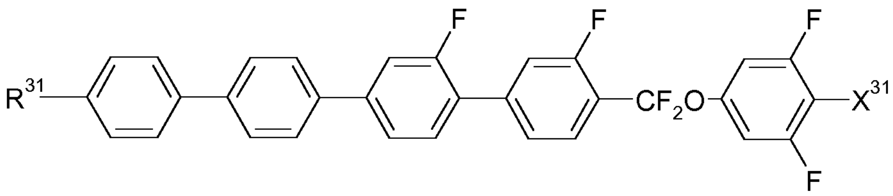

IIIC-3

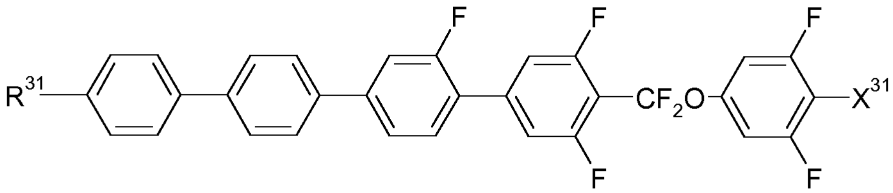

IIIC-4

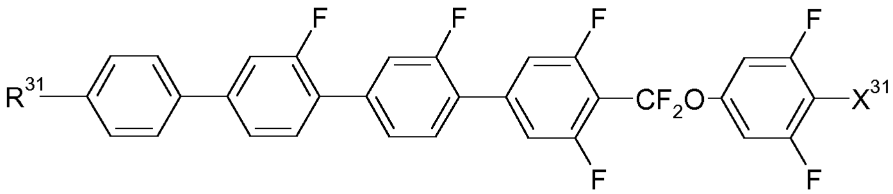

IIIC-5

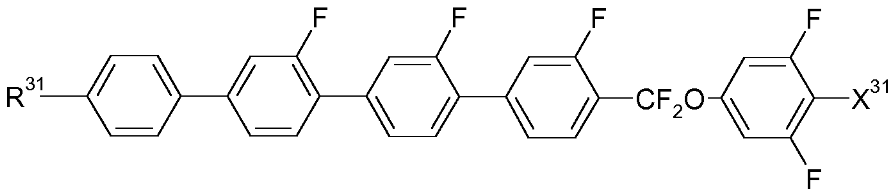

IIIC-6

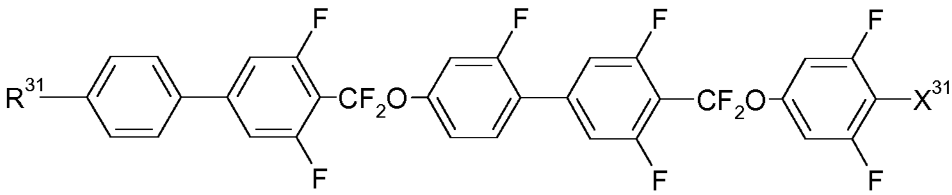

IIIC-7

wherein $R^{31}$ and $X^{31}$ have the meanings indicated above in formula III.

Especially preferred are compounds of formulae IIIC-1, IIIC-4 and IIIC-7.

The media in accordance with the present invention optionally comprise one or more compounds of the formula IV having three six-membered rings,

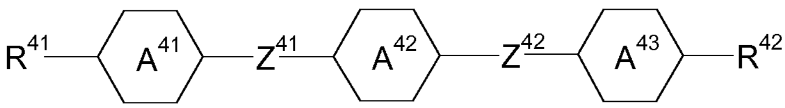

IV

wherein

$R^{41}$ and $R^{42}$, independently of one another, denote unfluorinated alkyl or alkoxy having 1 to 15, preferably 3 to 10, C atoms or unfluorinated alkenyl, alkenyloxy or alkoxyalkyl having 2 to 15, preferably 3 to 10, C atoms, preferably unfluorinated alkyl or alkenyl,

$Z^{41}$ and $Z^{42}$ independently of one another, denote $-CH_2CH_2-$, *trans*-CH=CH or a single bond, preferably one or more of them denotes a single bond, and particularly preferably all denote a single bond, and

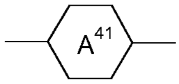 to 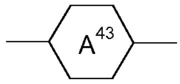,

independently of one another, denote

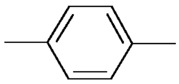, 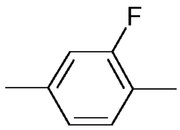, 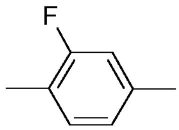,

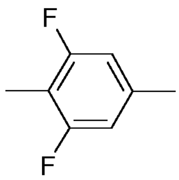, 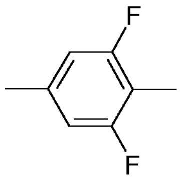, 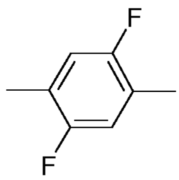,

or

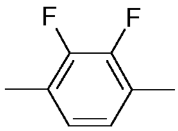,

preferably

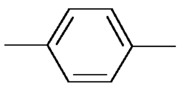, 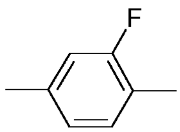, 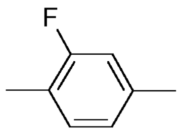,

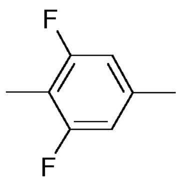, 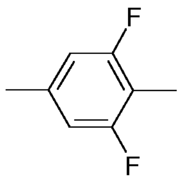

or

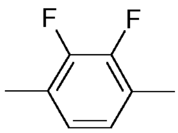.

Compounds of the formula IVA are preferably selected from the group of compounds of the formulae IVA-1 to IVA-7,

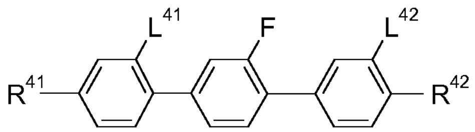

IVA-1

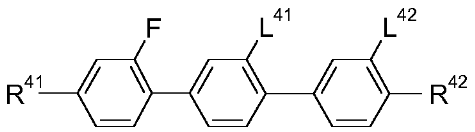

IVA-2

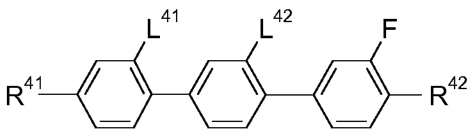

IVA-3

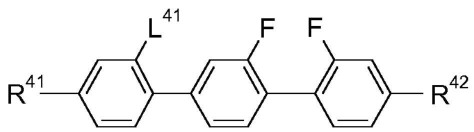

IVA-4

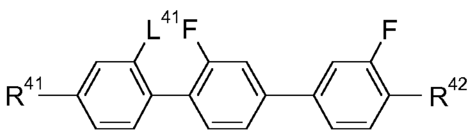

IVA-5

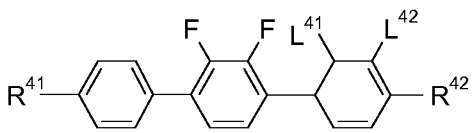

IVA-6

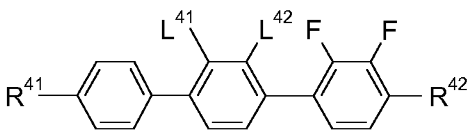

IVA-7

where in each case the compounds of the formula IVA-1 are excluded from the compounds of the formulae IVA-2 and IVA-3, the compounds of the formula IVA-2 are excluded from the compounds of the formula IVA-3 and the compounds of the formula IVA-6 are excluded from the compounds of the formula IVA-7, and

wherein the parameters $R^{41}$ and $R^{42}$ have the respective meanings indicated above for formula VI and

$L^{41}$ and $L^{42}$ each, independently of one another, denote H or F.

The preferred combinations of ($R^{41}$ and $R^{42}$) here are ($C_nH_{2n+1}$ and $C_mH_{2m+1}$), ($C_nH_{2n+1}$ and O-$C_mH_{2m+1}$), ($CH_2$=CH-$(CH_2)_Z$ and $C_mH_{2m+1}$), ($CH_2$=CH-$(CH_2)_Z$ and O-$C_mH_{2m+1}$), ($C_nH_{2n+1}$ and $(CH_2)_Z$-CH=$CH_2$), (($C_nH_{n+1}$)-CH=CH-$(CH_2)_Z$ and O-$C_mH_{2m+1}$) and (($C_nH_{2n+1}$ and $(CH_2)_Z$-CH=CH-($C_mH_{2m+1}$)), wherein

n and m denotes an integer in the range from 1 to 7 and preferably from 1 to 5, and

z denotes 0, 1, 2, 3 or 4, preferably 0 or 2.

Especially preferred compounds of formulae IVA-1 to IVA-7 are compounds of the formula IVA-1.

The media in accordance with the present invention optionally comprise one or more compounds of the formula V having four six-membered rings,

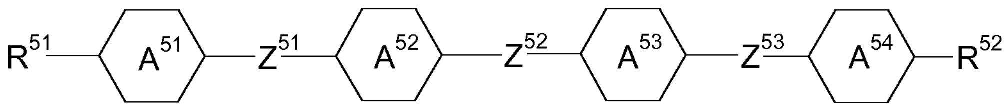

V

wherein

$R^{51}$ and $R^{52}$ independently of one another, denote unfluorinated alkyl or alkoxy having 1 to 15, preferably 3 to 10, C atoms or unfluorinated alkenyl, alkenyloxy or alkoxyalkyl having 2 to 15, preferably 3 to 10, C atoms, preferably unfluorinated alkyl or alkenyl,

$Z^{51}$ to $Z^{53}$ independently of one another, denote $-CH_2CH_2-$, *trans*--CH=CH- or a single bond, preferably one or more of them denotes a single bond, and particularly preferably all denote a single bond,

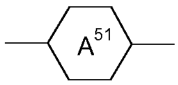

denotes 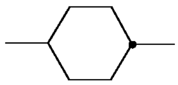 , 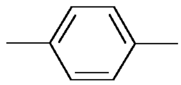 ,

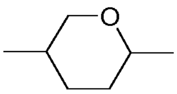 or 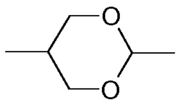 ,

and

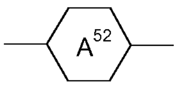 to 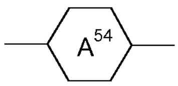

independently of one another, denote

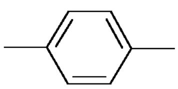 , 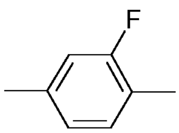 , 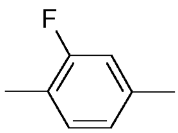 ,

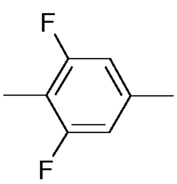 , 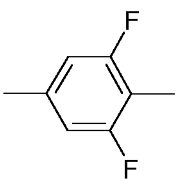 , 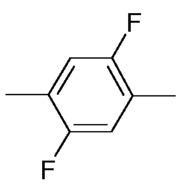

or

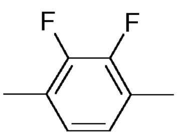 .

The compounds of the formula V are preferably selected from the group of compounds of the formulae VA to VC,

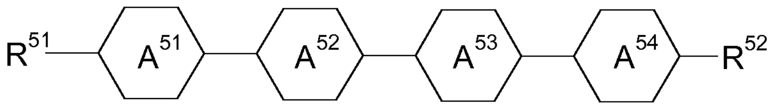

VA

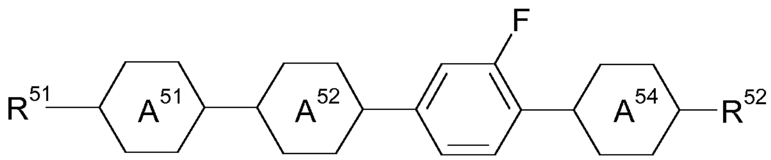

VB

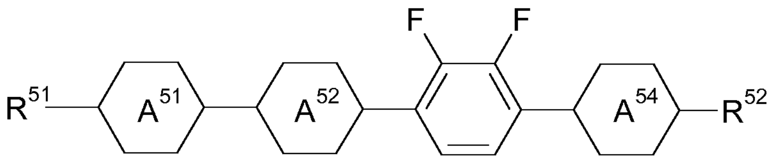

VC

wherein the parameters have the respective meanings indicated above in formula V and preferably one of

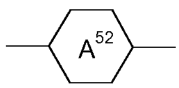

to

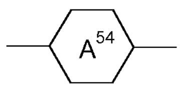

denotes

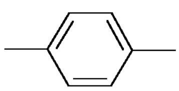

,

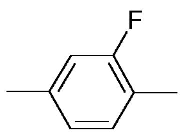

,

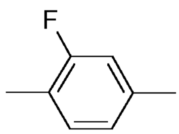

,

and

$R^{51}$ has the meaning indicated above in formula V and preferably denotes $C_nH_{2n+1}$ or $CH_2$=CH-$(CH_2)_Z$, and

$R^{52}$ has the meaning indicated above in formula V and preferably denotes $C_mH_{2m+1}$ or O-$C_mH_{2m+1}$ or $(CH_2)_Z$-CH=$CH_2$, and wherein

n and m independently of one another, denote an integer in the range from 1 to 7 and preferably from 1 to 5, and

z denotes 0, 1, 2, 3 or 4, preferably 0 or 2.

The preferred combinations of ($R^{51}$ and $R^{52}$) are, in particular, ($C_nH_{2n+1}$ and $C_mH_{2m+1}$) and ($C_nH_{2n+1}$ and O-$C_mH_{2m+1}$).

The compounds of the formula VA are preferably selected from the group of compounds of the formulae VA-1 to VA-6,

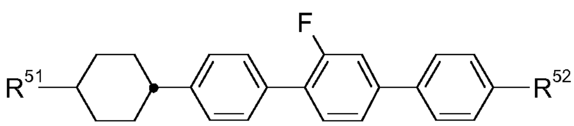

VA-1

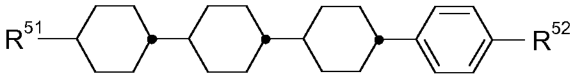

VA-2

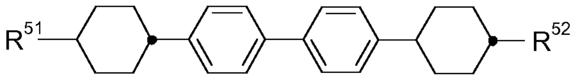

VA-3

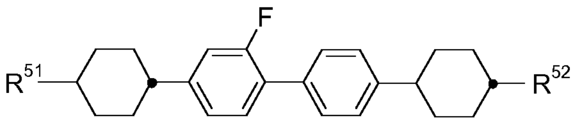

VA-4

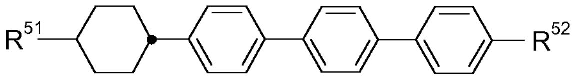

VA-5

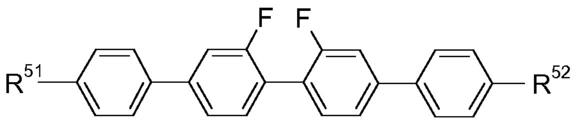

VA-6

wherein

$R^{51}$ has the meaning indicated above in formula V and preferably denotes $C_nH_{2n+1}$ or $CH_2$=CH-$(CH_2)_Z$,

$R^{52}$ has the meaning indicated above in formula V and preferably denotes $C_mH_{2m+1}$ or O-$C_mH_{2m+1}$ or $(CH_2)_Z$-CH=$CH_2$,

n and m independently of one another, denote an integer in the range from 1 to 7 and preferably from 1 to 5, and

z denotes 0, 1, 2, 3 or 4, preferably 0 or 2.

The preferred combination of ($R^{51}$ and $R^{52}$) are, in particular, ($C_nH_{2n+1}$ and $C_mH_{2m+1}$).

The compounds of formula VB are preferably selected from the group of compounds of the formulae VB-1 and VB-2,

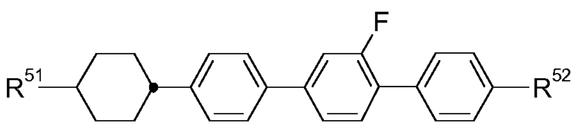

VB-1

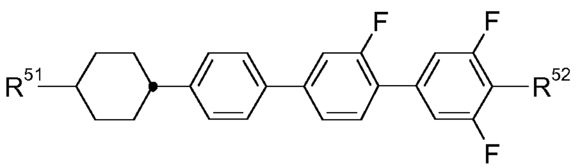

VB-2

wherein the parameters have the meanings given above in formula V and preferably

$R^{51}$ has the meaning indicated above in formula Vand preferably denotes $C_nH_{2n+1}$, and

n denotes an integer in the range from 1 to 7 and preferably from 1 to 5.

The compounds of the formula VC are preferably selected from compounds of the formulae VC-1,

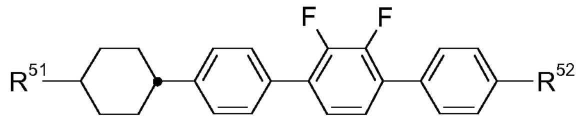

VC-1

wherein

$R^{51}$ has the meaning indicated above and preferably denotes $C_nH_{2n+1}$ or $CH_2$=CH-$(CH_2)_Z$,

$R^{52}$ has the meaning indicated above and preferably denotes $C_mH_{2m+1}$ or O-$C_mH_{2m+1}$ or $(CH_2)_Z$-CH=$CH_2$,

n and m independently of one another, denote an in the range from 1 to 7 and preferably from 1 to 5, and

z denotes 0, 1, 2, 3 or 4, preferably 0 or 2.

The preferred combinations of ($R^{51}$ and $R^{52}$) here are, in particular, ($C_nH_{2n+1}$ and $C_mH_{2m+1}$) and ($C_nH_{2n+1}$ and O-$C_mH_{2m+1}$), particularly preferably ($C_nH_{2n+1}$ and O-$C_mH_{2m+1}$).

Other mesogenic compounds, which are not explicitly mentioned above, can optionally and advantageously also be used in the media in accordance with the present invention. Such compounds are known to the person skilled in the art.

The compounds of formulae IA, IB and/or IC, II to V can be synthesized according to or in analogy to methods which are known per se and which are described in standard works of organic chemistry such as, for example, Houben-Weyl, Methoden der organischen Chemie, Thieme-Verlag, Stuttgart.

A preferred method of preparation of compounds of formula IB-1c is shown in the following synthesis scheme.

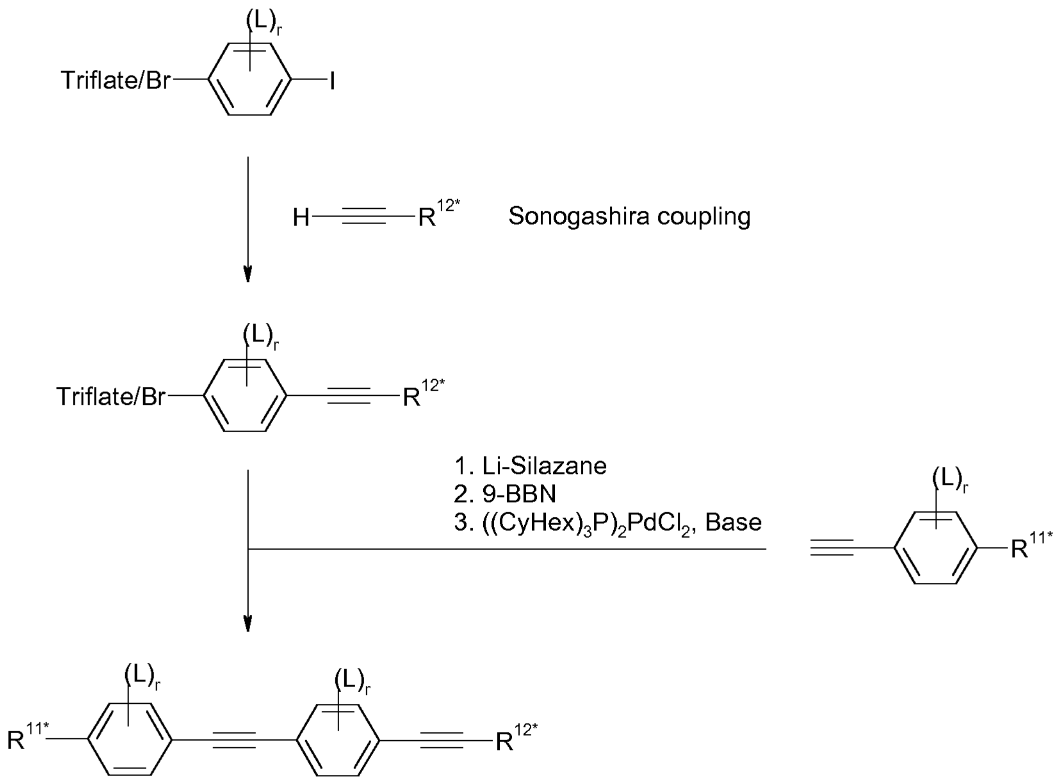


wherein

$R^{11*}$ has the meaning indicated above for $R^{11}$ in formula IB and preferably denotes $C_nH_{2n+1}$,

$R^{12*}$ has the meaning indicated above for $R^{12}$ in formula IB and preferably denotes $C_nH_{2n+1}$,

n denotes an integer in the range from 1 to 7 and preferably from 1 to 5,

L has, each and independently to one another in each occurrence one of the meanings of $R^{11*}$ different from H,

r denotes, each and independently to one another in each occurrence 0, 1, 2, 3 or 4.

A preferred method of preparation of compounds of formula IB-1e is shown in the following synthesis scheme.

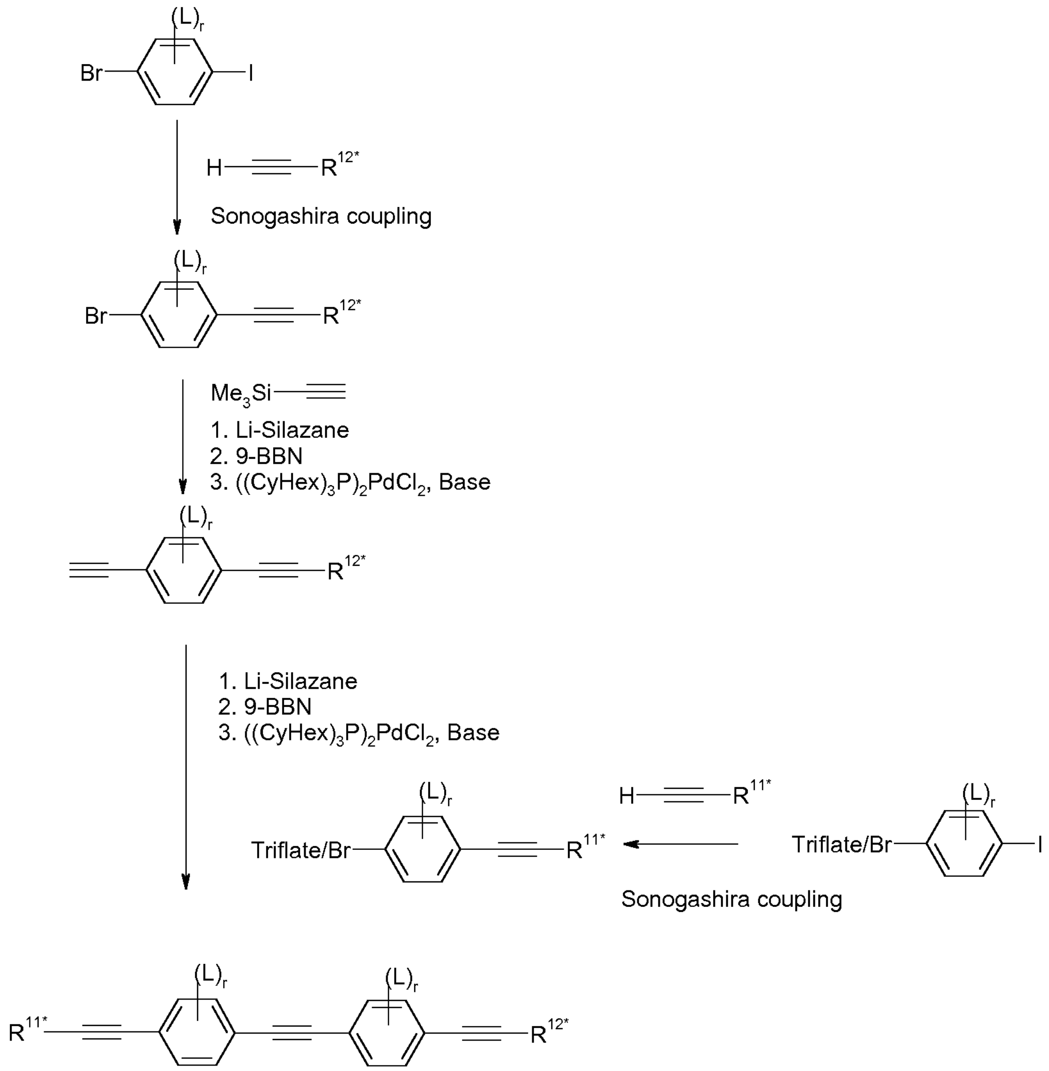


wherein

$R^{11*}$ has the meaning indicated above for $R^{11}$ in formula IB and preferably denotes $C_nH_{2n+1}$,

$R^{12*}$ has the meaning indicated above for $R^{12}$ in formula IB and preferably denotes $C_nH_{2n+1}$,

n denotes an integer in the range from 1 to 7 and preferably from 1 to 5,

L has, each and independently to one another in each occurrence one of the meanings of $R^{11*}$ different from H,

r denotes, each and independently to one another in each occurrence 0, 1, 2, 3 or 4.

The liquid-crystalline media according to the invention consist of a plurality of compounds, preferably 3 to 20, more preferably 3 to 15 and more preferably 3 to 10 compounds. These compounds are mixed in a conventional manner. In general, the desired amount of the compound used in the smaller amount is dissolved in the compound used in the larger amount. If the temperature is above the clearing point of the compound used in the higher concentration, it is particularly easy to observe completion of the dissolution process. It is, however, also possible to prepare the media in

other conventional ways, for example using so-called pre-mixes, which can be, for example, homologous or eutectic mixtures of compounds, or using so-called "multibottle" systems, the constituents of which are themselves ready-to-use mixtures.

In an even more preferred embodiment, the liquid-crystalline media comprise one or more compounds of the formula IB, one or more compounds of the formula II, one or more compounds of the formula III and one or more compounds of the formula IV.

In an especially preferred embodiment, the liquid-crystalline media comprise one or more compounds of the formula IB, one or more compounds of the formula II, one or more compounds of the formula III, one or more compounds of the formula IV and one or more compounds of the formula V.

In particular, preference are given to the liquid-crystalline media, which comprise one or more compounds of the formula IB and one or more compounds of the formula II, and/or one or more compounds of the formula III, and/or one or more compounds of the formula IV and/or one or more compounds of the formula V.

In a preferred embodiment, the liquid-crystalline media comprises one, two, three or more compounds of formula IB.

The liquid-crystalline media in accordance with the present application preferably comprise

- in total 5 to 60 %, preferably 7 to 50 % and particularly preferably 10 to 40 % of compounds of the formula IB, and
- optionally, in total 1 to 20 %, preferably 3 to 15 % and particularly preferably 5 to 10 % of compounds of the formula IA, and/or
- optionally in total 3 to 50 %, preferably 4 to 45 % and particularly preferably 5 to 35 % of compounds of the formula IC, and
- in total 10 to 80 %, preferably 12 to 70 %, particularly preferably 15 to 60 % of compounds of the formula II, and
- in total 0 to 30 %, preferably 1 to 25 % and particularly preferably 5 to 20 % of compounds of the formula III, and
- optionally in total 0 to 50 %, preferably 3 to 45 % and particularly preferably 5 to 40 % of compounds of the formula IV and/or V, but all together in a total amount $\leq$ 100 %.

Further preferred are liquid-crystalline media comprising,

- 5 to 80 %, more preferably 10 to 75 %, even more preferably 15 to 65 % in total of the whole mixture of one or more compounds of formula IB and formulae IA, and/or IC, preferably selected from compounds of formulae IA-2 and IC-I to IC-3, and
- 10 to 80 %, preferably 12 to 70 %, particularly preferably 15 to 60 %, in total of the whole mixture of one or more compounds of formula II, preferably selected from compounds of formulae IIA to IIE, more preferably selected from compounds of formulae IIC and/or IID,

and/or

- optionally 0 to 50 %, more preferably 0 to 45 %, even more preferably 0 to 40 % in total of the whole mixture compounds of the formulae III to V.

but in a total amount of the total mixture $\leq$ 100 %.

Especially preferred concentrations of compounds of formulae III to V are:

- 1 to 30 %, preferably 3 to 25 % and particularly preferably 5 to 20 %, in total of the whole mixture of one or more compounds of the formula III, preferably selected from compounds of formulae IIIA to IIIC, and/or
- 1 to 40 %, preferably 3 to 35 % and particularly preferably 5 to 30 %, in total of the whole mixture of one or more compounds of the formula IV, preferably selected from compounds of formulae IVA-1 to IVA-7, more preferably selected from compounds of formula IVA-1,

and /or

- 1 to 40 %, preferably 3 to 35 % and particularly preferably 5 to 30 %, in total of the whole mixture of one or more

compounds of the formula V, preferably selected from compounds of formulae VA to VC more preferably selected from compounds of formulae VA-3 and/or VB-1,

but in a total amount of the total mixture ≤ 100 %.

In particular, the liquid-crystalline media according to the present invention consist exclusively of the above-mentioned compounds.

In this application, "comprise" in connection with compositions means that the entity in question, i.e. the medium comprises the compound or compounds indicated, preferably in a total concentration of 3 % or more and more preferably 5 % or more. Additionally, "consist exclusively" means that the entity in question comprises preferably 99 % or more and more preferably 100.0 % of the compound or compounds indicated.

The liquid-crystalline media according to the present invention may contain further additives, like dyes, antioxidants, chiral dopants, UV stabilizers, in usual concentrations. The total concentration of these further constituents is in the range of 50 ppm to 10 %, preferably 100 ppm to 6 %, based on the total mixture. The concentrations of the individual compounds used each are preferably in the range of 0.1 % to 3 %.

The liquid-crystalline media in accordance with the present invention preferably have a clearing point of 80°C or more, more preferably 90°C or more, even more preferably 100°C or more, most preferably 120°C or more but having crystallisation points of - 20°C or lower, more preferably - 30°C or lower, even more preferably - 40°C or lower. Most preferably, all limits are independently combined with each other.

Most preferably, the liquid-crystalline media according to the invention preferably have nematic phases of in each case at least from -20°C to 80°C, preferably from -30°C to 100°C and more particularly preferably from -40°C to 120°C. Most preferably, all limits are independently combined with each other. The expression have a nematic phase here means on the one hand that no smectic phase and no crystallisation are observed at low temperatures at the corresponding temperature and on the other hand that no clearing occurs on heating from the nematic phase.

The $\Delta\varepsilon$ of the liquid-crystalline media in accordance with the invention, at 1 kHz and 20°C, are preferably 1 or more, more preferably 2 or more and more preferably 3 or more.

In the present application, the expression dielectrically positive describes compounds or components where $\Delta\varepsilon > 3.0$, dielectrically neutral describes those where $-1.5 \leq \Delta\varepsilon \leq 3.0$ and dielectrically negative describes those where $\Delta\varepsilon < -1.5$. $\Delta\varepsilon$ is determined at a frequency of 1 kHz and at 20°C. The dielectric anisotropy of the respective compound is determined from the results of a solution of 10 % of the respective individual compound in a nematic host mixture. If the solubility of the respective compound in the host mixture is less than 10 %, the concentration is reduced to 5%. The capacitances of the test mixtures are determined both in a cell having homeotropic alignment and in a cell having homogeneous alignment. The cell thickness of both types of cells is approximately 20 μm. The voltage applied is a rectangular wave having a frequency of 1 kHz and an effective value of typically 0.5 V to 1.0 V, but it is always selected to be below the capacitive threshold of the respective test mixture.

$\Delta\varepsilon$ is defined as $(\varepsilon_{||} - \varepsilon_{\perp})$, while $\varepsilon_{ave.}$ is $(\varepsilon_{||} + 2\,\varepsilon_{\perp})/3$.

The host mixture used for dielectrically positive compounds is mixture ZLI-4792 and that used for dielectrically neutral and dielectrically negative compounds is mixture ZLI-3086, both from Merck KGaA, Germany. The absolute values of the dielectric constants of the compounds are determined from the change in the respective values of the host mixture on addition of the compounds of interest. The values are extrapolated to a concentration of the compounds of interest of 100 %.

The $\Delta n$ of the liquid-crystalline media in accordance with the present invention, at 589 nm ($Na^D$) and 20°C, is preferably in the range from 0.230 or more to 0.90 or less, more preferably in the range from 0.240 or more to 0.90 or less, even more preferably in the range from 0.250 or more to 0.85 or less. Most preferably, all limits are independently combined with each other.

Furthermore, the liquid-crystalline media according to the invention are characterised by high dielectric anisotropy values in the microwave range at 19 GHz is, for example, 0.50 or more, preferably 0.60 or more, more preferably 0.70 or more. Most preferably, all limits are independently combined with each other.

The liquid-crystalline media are investigated with respect to their properties in the microwave frequency range as described in A. Penirschke, S. Müller, P. Scheele, C. Weil, M. Wittek, C. Hock and R. Jakoby: "Cavity Perturbation Method for Characterization of Liquid Crystals up to 35 GHz", 34th European Microwave Conference - Amsterdam, pp. 545-548.

Compare in this respect also A. Gaebler, F. Golden, S. Müller, A. Penirschke and R. Jakoby "Direct Simulation of Material Permittivites ...", 12MTC 2009 - International Instrumentation and Measurement Technology Conference, Singapore, 2009 (IEEE), pp. 463-467, and DE 10 2004 029 429 A, wherein a measurement method is likewise described in detail.

The liquid crystal is introduced into a polytetrafluoroethylene (PTFE) or fused silica capillary. The capillary has an internal radius of 180 μm and an external radius of 350 μm. The effective length is 2.0 cm. The filled capillary is introduced into the centre of the cavity with a resonance frequency of 30 GHz. This cavity has a length of 6.6 mm, a

width of 7.1 mm and a height of 3.6 mm. The input signal (source) is then applied, and the result of the output signal is recorded using a commercial vector network analyser. For other frequencies (e.g. 19 GHz), the dimensions of the cavity can be adjusted accordingly.

The change in the resonance frequency and the Q factor between the measurement with the capillary filled with the liquid crystal and the measurement without the capillary filled with the liquid crystal is used to determine the dielectric constant and the loss angle at the corresponding target frequency by means of equations 10 and 11 in A. Penirschke, S. Müller, P. Scheele, C. Weil, M. Wittek, C. Hock and R. Jakoby: "Cavity Perturbation Method for Characterization of Liquid Crystals up to 35GHz", 34th European Microwave Conference - Amsterdam, pp. 545-548, as described therein.

The values for the components of the properties perpendicular and parallel to the director of the liquid crystal are obtained by alignment of the liquid crystal in a magnetic field. To this end, the magnetic field of a permanent magnet is used. The strength of the magnetic field is 0.35 tesla. The alignment of the magnets is set correspondingly and then rotated correspondingly through 90°.

The rotational viscosity ($\gamma_1$) of the preferred liquid-crystalline media are, preferably in combination with the above indicated ranges for the nematic phase, ≤ to 600 mPa·s, preferably ≤ 400 mPa·s, more preferably ≤ 300 mPa·s but ≥ 50 mPa·s, preferably ≥100 mPa·s and more preferably ≥ 150 mPa·s. Most preferably, all limits are independently combined with each other.

The tunability ($\tau$) of the liquid-crystalline media are preferably in combination with the above indicated ranges for the rotational viscosity ≥ 0.15, preferably ≥ 0.17, more preferably ≥ 0.18, but ≤ 0.35, preferably ≤ 0.30 and more preferably ≤ 0.25. Most preferably, all limits are independently combined with each other.

The material quality ($\eta$) of the preferred liquid-crystalline materials are ≥8, preferably ≥ 9, more preferably ≥ 10, but ≤ 35, preferably ≤ 30 and more preferably ≤ 25. Most preferably, all limits are independently combined with each other.

Preferably the preferred ranges for T(N,I), $\gamma_1$, $\tau$ and $\eta$ are combined with each other.

The liquid crystalline media according to the present invention are very well suited for the preparation of microwave components, such as tunable phase shifters. These may be tuned by the application of magnetic and/or electric fields. Tuning by electric fields is generally preferred. These phase shifters are operable in the UHF-band (0.3-1GHz), L-band (1-2GHz), S-band (2-4GHz), C-band (4-8GHz), X-band (8-12GHz), Ku-band (12-18GHz), K-band (18-27GHz), Ka-band (27-40GHz), V-band (50-75GHz), W-band (75-110GHz) and up to 1THz.

Preferable frequencies for operation are C-band, X-band, Ku-band, K-band, Ka-band, V-band, W-band, and up to 1THz. Particularly preferable frequencies for operation are Ku-band, K-band, Ka-band, V-band, W-band, and up to 1THz.

The construction of the phase shifters according to the present application is known to the expert. Typically loaded line phase shifters, "inverted microstrip lines" (short IMSL), Finline phase shifters, preferably Antipodal Finline phase shifters, slotline phase shifters, microstrip line phase shifters or coplanar waveguides (CPW) phase shifters are used. These components allow the realization of reconfigurable antenna arrays, which are fully electrically reconfigurable and which allow to steer the main beam direction of the antennas, to blank out interferers and/or to achieve high directivity. Another preferred embodiment is waveguide partially filled with the liquid crystals according to the present invention, as described in WO 2011/036243 A1, which is encompassed herein by reference herewith.

In a preferred embodiment the inventive phase shifters are combined into array antennas, preferably into phased array antennas, reflectarray antennas, and arrays consisting of Vivaldi antennas.

Especially preferred applications for the tunable antenna arrays according to the present application are satellite communication systems, for operation e.g. between satellites, from satellites to ground stations, from mobile ground stations via satellite to stationary ground stations or to other mobile ground stations, e.g. for receiving and sending communication, television or video to vehicles like ships, planes, trains and cars. Other preferred applications are short-distance antennas of the wireless routers to the laptop PCs, tablet computers and mobile devices.

For an overview of terms and definitions in connection with liquid crystals and mesogens see Pure Appl. Chem. 73(5), 888 (2001) and C. Tschierske, G. Pelzl and S. Diele, Angew. Chem. 2004, 116, 6340-6368.

In the present application, the term "compounds" is taken to mean both one compound and a plurality of compounds, unless expressly stated otherwise.

The term "mesogenic group" means a group with the ability to induce liquid crystal (LC) phase behaviour. The compounds comprising mesogenic groups do not necessarily have to exhibit an LC phase themselves. It is also possible that they show LC phase behaviour only in mixtures with other compounds (e.g. liquid-crystalline host mixture), or when the mesogenic compounds or the mixtures thereof are polymerised. For the sake of simplicity, the term "liquid crystal" is used hereinafter for both mesogenic and LC materials.

The parameter ranges indicated in this application all include the limit values, unless expressly stated otherwise.

Throughout this application, the following conditions and definitions apply, unless expressly stated otherwise. All concentrations are quoted in per cent by weight and relate to the respective mixture as a whole, all temperatures are

quoted in degrees Celsius and all temperature differences are quoted in differential degrees. All physical properties are determined in accordance with "Merck Liquid Crystals, Physical Properties of Liquid Crystals", Status Nov. 1997, Merck KGaA, Germany, and are quoted for a temperature of 20°C, unless expressly stated otherwise. The optical anisotropy ($\Delta n$) is determined at a wavelength of 589.3 nm. The dielectric anisotropy ($\Delta\varepsilon$) is determined at a frequency of 1 kHz or if explicitly stated at a frequency 19 GHz.

The term "alkyl" preferably encompasses straight chain and branched alkyl groups having 1 to 15 carbon atoms, in particular the straight chain groups methyl, ethyl, propyl, butyl, pentyl, hexyl and heptyl. Groups having 2 to 10 carbon atoms are generally preferred.

The term "alkenyl" preferably encompasses straight chain and branched alkenyl groups having 2 to 15 carbon atoms, in particular the straight-chain groups. Particularly preferred alkenyl groups are $C_2$- to $C_7$-1E-alkenyl, $C_4$- to $C_7$-3E-alkenyl, $C_5$- to $C_7$-4-alkenyl, $C_6$- to $C_7$-5-alkenyl and $C_7$-6-alkenyl, in particular $C_2$- to $C_7$-1E-alkenyl, $C_4$- to $C_7$-3E-alkenyl and $C_5$- to $C_7$-4-alkenyl. Examples of further preferred alkenyl groups are vinyl, 1E-propenyl, 1E-butenyl, 1E-pentenyl, 1E-hexenyl, 1E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 carbon atoms are generally preferred.

The term "fluoroalkyl" preferably encompasses straight-chain groups having terminal fluorine, i.e. fluoromethyl, 2-fluoroethyl, 3-fluoropropyl, 4-fluorobutyl, 5-fluoropentyl, 6-fluorohexyl and 7-fluoroheptyl. However, other positions of the fluorine are not excluded.

The term "oxaalkyl" or "alkoxyalkyl" preferably encompasses straight-chain radicals of the formula $C_nH_{2n+1}$-O-$(CH_2)_m$, wherein n and m each, independently of one another, denote 1 to 10. Preferably, n is 1 and m is 1 to 6.

All temperatures, such as, for example, the melting point T(C,N) or T(C,S), the transition from the smectic (S) to the nematic (N) phase T(S,N) and the clearing point T(N,I) of the liquid crystals, are quoted in degrees Celsius. All temperature differences are quoted in differential degrees.

In the present invention and especially in the following examples, the structures of the mesogenic compounds are indicated by means of abbreviations also referred to as acronyms. In these acronyms, the chemical formulae are abbreviated as follows using Tables A to C below. All groups $C_nH_{2n+1}$, $C_mH_{2m+1}$ and $C_lH_{2l+1}$ or $C_nH_{2n-1}$, $C_mH_{2m-1}$ and $C_lH_{2l-1}$ denote straight-chain alkyl or alkenyl, preferably 1-E-alkenyl, respectively, in each case having n, m or l C atoms. Table A lists the codes used for the ring elements of the core structures of the compounds, while Table B shows the linking groups. Table C gives the meanings of the codes for the left-hand or right-hand end groups. Table D shows illustrative structures of compounds with their respective abbreviations.

**Table A: Ring elements**

| | | | |
|---|---|---|---|
| **C** | 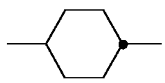 | | |
| **D** | 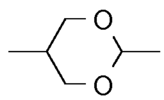 | **DI** | 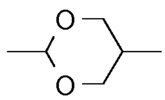 |
| **A** | 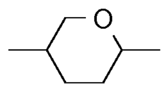 | **AI** | 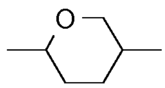 |
| **P** | 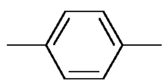 | | |
| **G** | 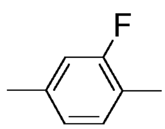 | **GI** | 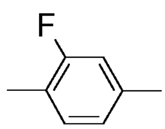 |
| **U** | 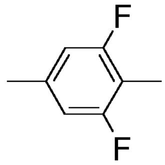 | **UI** | 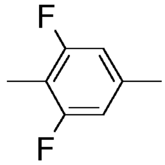 |

(continued)

| | | | |
|---|---|---|---|
| **Y** | 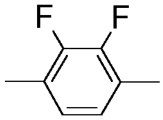 | | |
| **M** | 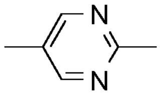 | **MI** | 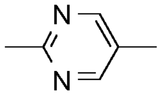 |
| **N** | 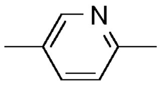 | **NI** | 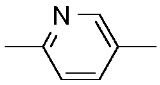 |
| **Np** | 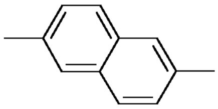 | | |
| **N3f** | 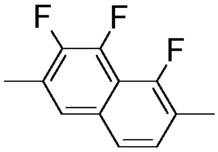 | **N3fI** | 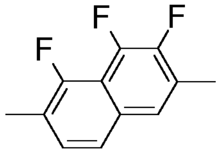 |
| **tH** | 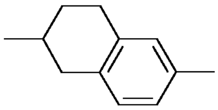 | **tHI** | 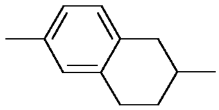 |
| **tH2f** | 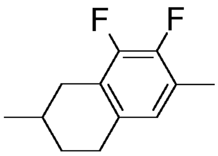 | **tH2fI** | 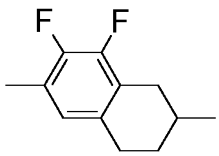 |
| **dH** | 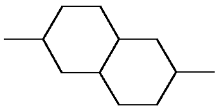 | | |
| **K** | 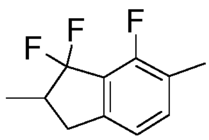 | **KI** | 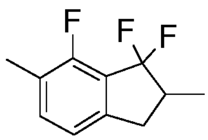 |
| **L** | 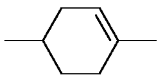 | **LI** | 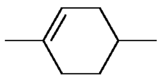 |
| **F** | 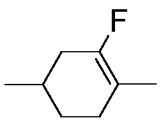 | **FI** | 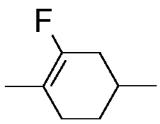 |

**Table B: Linking groups**

| | | | |
|---|---|---|---|
| **E** | $-CH_2CH_2-$ | **Z** | -CO-O- |
| **V** | -CH=CH- | **ZI** | -O-CO- |
| **X** | -CF=CH- | **O** | $-CH_2-O-$ |
| **XI** | -CH=CF- | **OI** | $-O-CH_2-$ |
| **B** | -CF=CF- | **Q** | $-CF_2-O-$ |
| **T** | -C≡C- | **QI** | $-O-CF_2-$ |

(continued)

| | |
|---|---|
| **W** | $-CF_2CF_2-$ |

**Table C: End groups**

| **Left-hand side** | | **Right-hand side** | |
|---|---|---|---|
| | **Use alone** | | |
| **-n-** | $C_nH_{2n+1}-$ | **-n** | $--C_nH_{2n+1}$ |
| **-nO-** | $C_nH_{2n+1}-O-$ | **-nO** | $-O-C_nH_{2n+1}$ |
| **-V-** | $CH_2=CH-$ | **-V** | $-CH=CH_2$ |
| **-nV-** | $C_nH_{2n+1}-CH=CH-$ | **-nV** | $-C_nH_{2n}-CH=CH_2$ |
| **-Vn-** | $CH_2=CH- C_nH_{2n+1}-$ | **-Vn** | $-CH=CH-C_nH_{2n+1}$ |
| **-nVm-** | $C_nH_{2n+1}-CH=CH-C_mH_{2m}-$ | **-nVm** | $-CnH_{2n}-CH=CH-C_mH_{2m+1}$ |
| **-N-** | N≡C- | **-N** | -C≡N |
| **-S-** | S=C=N- | **-S** | -N=C=S |
| **-F-** | F- | **-F** | -F |
| **-CL-** | Cl- | **-CL** | -Cl |
| **-M-** | $CFH_2-$ | **-M** | $-CFH_2$ |
| **-D-** | $CF_2H-$ | **-D** | $-CF_2H$ |
| **-T-** | $CF_3-$ | **-T** | $-CF_3$ |
| **-MO-** | $CFH_2O-$ | **-OM** | $-OCFH_2$ |
| **-DO-** | $CF_2HO-$ | **-OD** | $-OCF_2H$ |
| **-TO-** | $CF_3O-$ | **-OT** | $-OCF_3$ |
| **-OXF-** | $CF_2=CH-O-$ | **-OXF** | $-O-CH=CF_2$ |
| **-A-** | H-C≡C- | **-A** | -C≡C-H |
| **-nA-** | $C_nH_{2n+1}$-C≡C- | **-An** | -C≡C-$C_nH_{2n+1}$ |
| **-NA-** | N≡C-C≡C- | **-AN** | -C≡C-C≡N |
| | **Use together with others** | | |
| **-...A...-** | -C≡C- | **-...A...** | -C≡C- |
| **-...V...-** | CH=CH- | **-...V...** | -CH=CH- |
| **-...Z...-** | -CO-O- | -...Z... | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

wherein n and m each denote integers and the three dots "..." are place-holders for other abbreviations from this table.

The following table shows illustrative structures together with their respective abbreviations. These are shown in order to illustrate the meaning of the rules for the abbreviations. They furthermore represent compounds, which are preferably used.

**Table D: Illustrative structures**

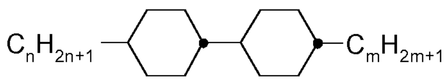

**CC-n-m**

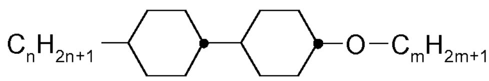

**CC-n-Om**

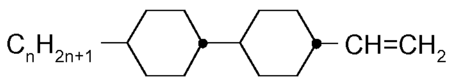

**CC-n-V**

(continued)

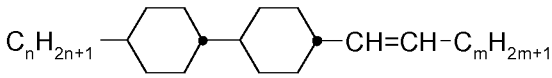

**CC-n-Vm**

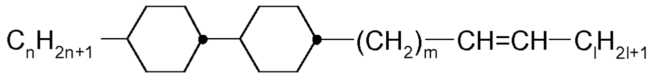

**CC-n-mVl**

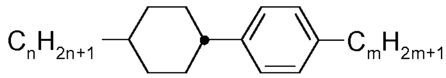

**CP-n-m**

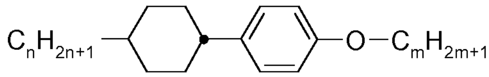

**CP-n-Om**

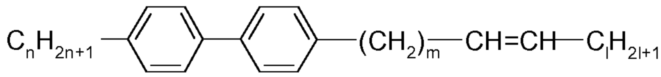

**PP-n-mVl**

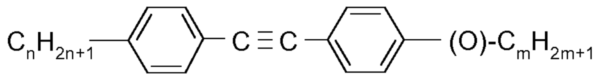

**PTP-n-(O)m**

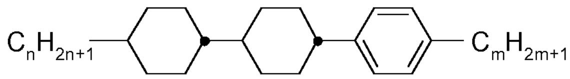

**CCP-n-m**

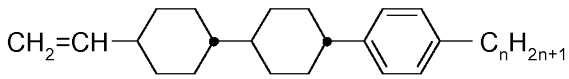

**CCP-V-n**

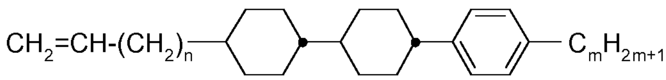

**CCP-Vn-m**

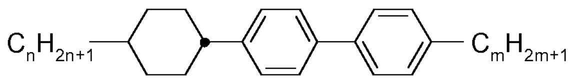

**CCP-n-m**

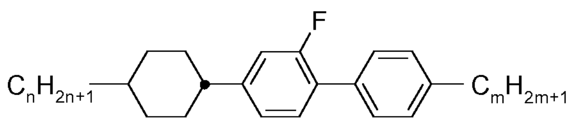

**CGP-n-m**

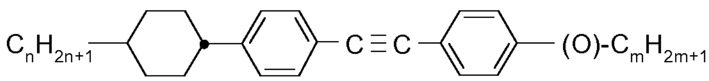

**CPTP-n-(O)m**

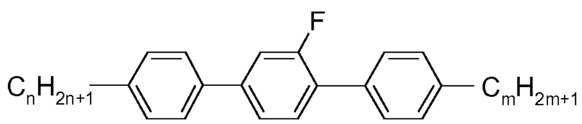

**PGP-n-m**

(continued)

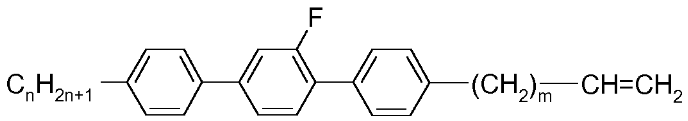

**PGP-n-mV**

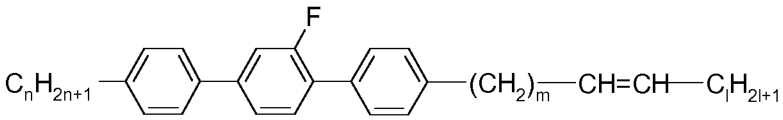

**PGP-n-mVI**

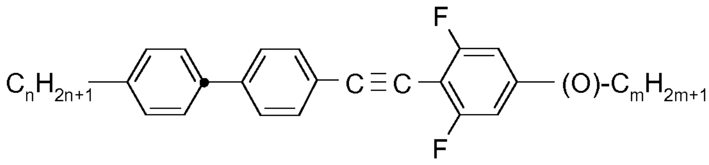

**PPTUI-n-(O)m**

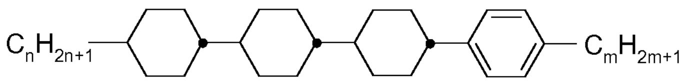

**CCCP-n-m**

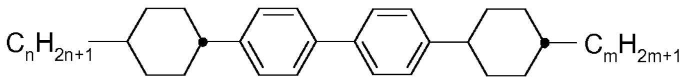

**CPPC-n-m**

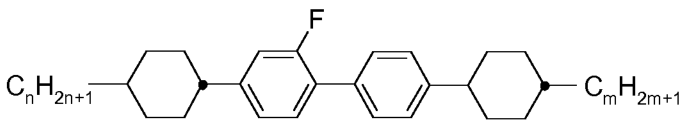

**CGPC-n-m**

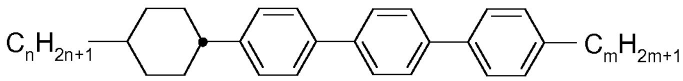

**CPPP-n-m**

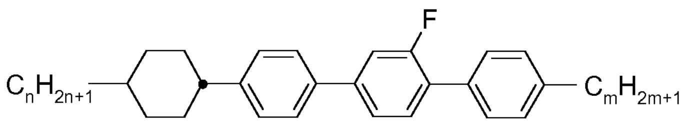

**CPGP-n-m**

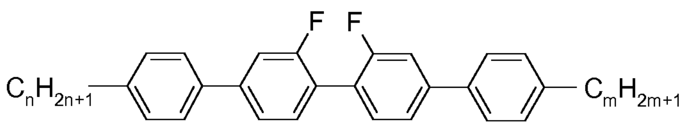

**PGGIP-n-m**

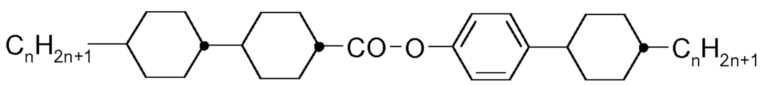

**CCZPC-n-m**

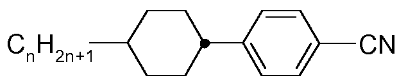

**CP-n-N**

(continued)

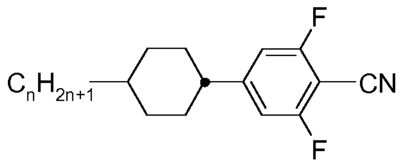

**CU-n-N**

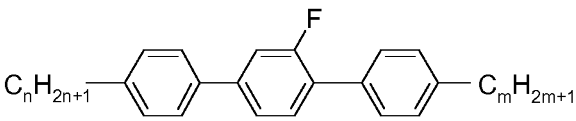

**PGIP-n-N**

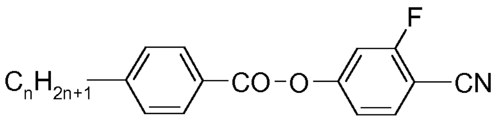

**PZG-n-N**

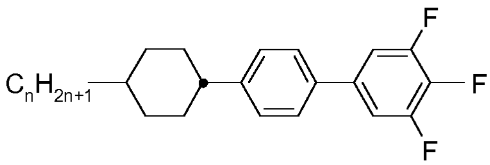

**CPU-n-F**

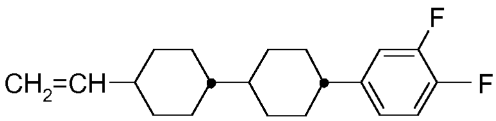

**CCG-V-F**

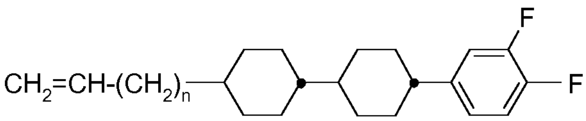

**CCG-Vn-F**

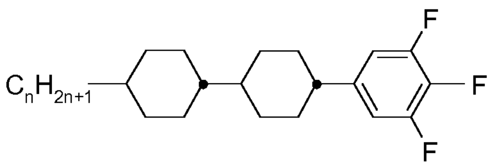

**CCU-n-F**

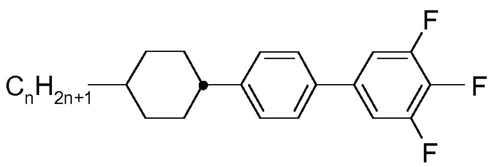

**CPU-n-F**

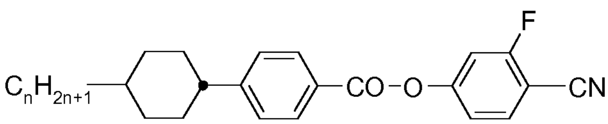

**CPZG-n-N**

(continued)

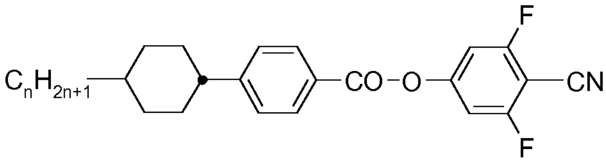

**CPZU-n-N**

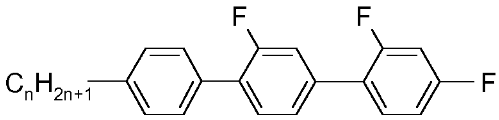

**PGIGI-n-F**

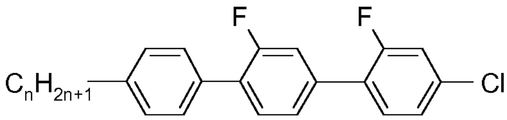

**PGIGI-n-CL**

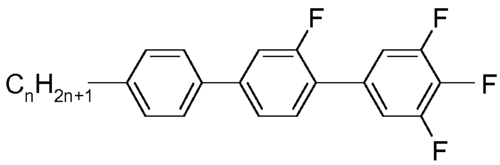

**PGU-n-F**

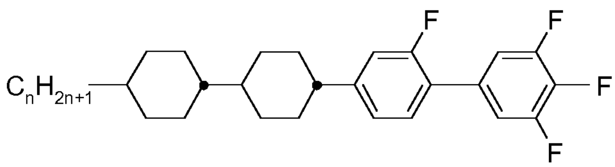

**CCGU-n-F**

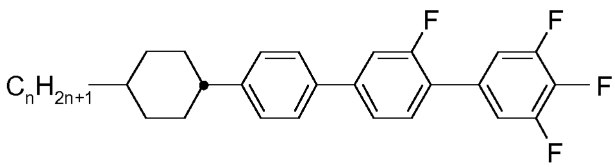

**CPGU-n-F**

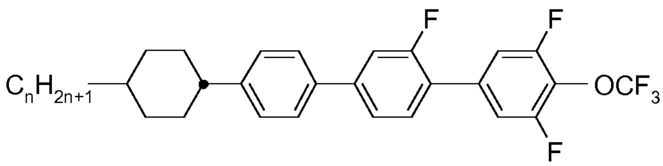

**CPGU-n-OT**

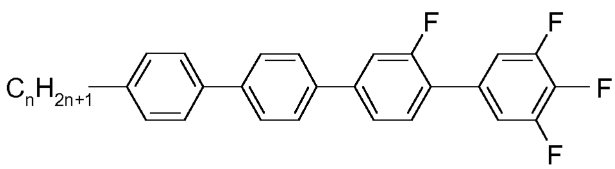

**PPGU-n-F**

(continued)

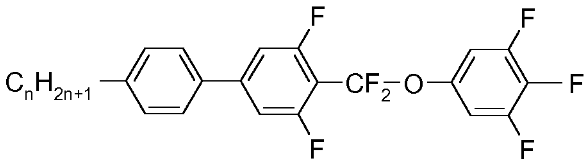

**PUQU-n-F**

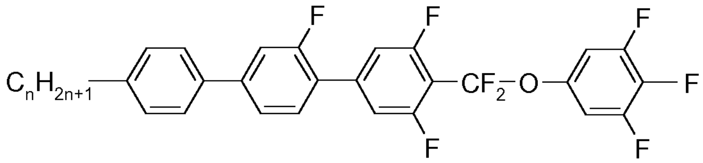

**PGUQU-n-F**

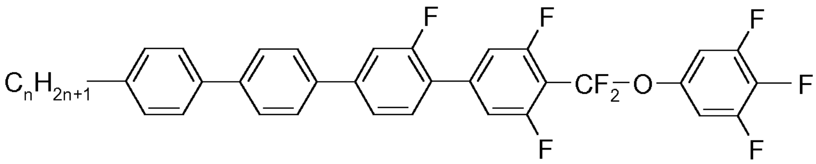

**PPGUQU-n-F**

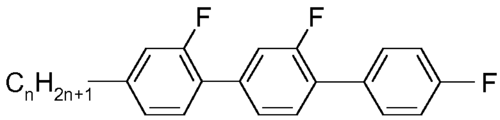

**GGP-n-F**

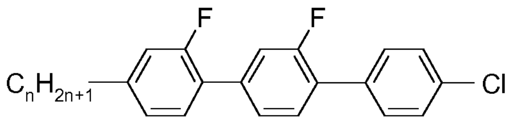

**GGP-n-CL**

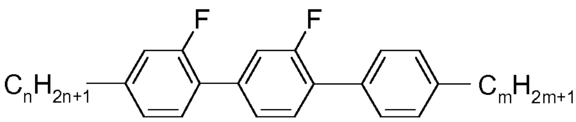

**GGP-n-m**

## Examples

The following examples illustrate the present invention without limiting it in any way.

However, it is clear to the person skilled in the art from the physical properties what properties can be achieved and in what ranges they can be modified. In particular, the combination of the various properties that can preferably be achieved is thus well defined for the person skilled in the art.

## Example 1 (Comparative)

A liquid-crystalline mixture M-1 having the composition and properties as indicated in the following table is prepared.

| Composition | | | Physical Properties | | |
|---|---|---|---|---|---|
| Compound No. | Abbreviation | Conc. / mass-% | T(N, I) | = 103 | °C |
| 1 | PPTUI-3-2 | 10.0 | $\Delta\varepsilon$(20°C, 1 kHz) | = 2.7 | |
| 2 | PPTUI-3-4 | 15.0 | $\gamma_1$ (20°C) | = 336 | mPa · s |

(continued)

| Composition | | | Physical Properties | |
|---|---|---|---|---|
| Compound | | Conc. | | |
| 3 | PPTUI-4-4 | 25.0 | tan $\delta_{\varepsilon\ r,\perp}$ (20°C, 19 GHz) | = 0.0130 |
| 4 | GGP-5-CL | 20.0 | tan $\delta_{\varepsilon\ r,\parallel}$ (20°C, 19 GHz) | = 0.0043 |
| 5 | PTP-3-5 | 15.0 | $\tau$ (20°C, 19 GHz) | = 0.229 |
| 6 | PTP-4-5 | 15.0 | $\eta$ (20°C, 19 GHz) | = 18.0 |
| $\Sigma$ | | 100.0 | | |

Example 2 (Comparative)

A liquid-crystalline mixture M-2 having the composition and properties as indicated in the following table is prepared.

| Composition | | | Physical Properties | | |
|---|---|---|---|---|---|
| Compound No. | Abbreviation | Conc. / mass-% | T(N, I) | = 128 | °C |
| 1 | PZG-4-N | 5.0 | $\Delta$n(20°C,589.3 nm) | = 0.289 | |
| 2 | PPTUI-3-2 | 20.0 | $\Delta\varepsilon$(20°C, 1 kHz) | = 5.2 | |
| 3 | PPTUI-3-4 | 33.0 | $\gamma_1$ (20°C) | = 317 | mPa · s |
| 4 | PTP-3-5 | 5.0 | tan $\delta_{\varepsilon r,\perp}$ (20°C, 19 GHz) | = 0.0155 | |
| 5 | PTP-4-5 | 5.0 | tan $\delta_{\varepsilon r,\parallel}$ (20°C, 19 GHz) | = 0.0048 | |
| 6 | CC-3-V | 10.0 | $\tau$ (20°C, 19 GHz) | = 0.218 | |
| 7 | GGP-3-CL | 5.0 | $\eta$ (20°C, 19 GHz) | = 14.0 | |
| 8 | GGP-5-CL | 10.0 | | | |
| 9 | CPGP-5-2 | 3.5 | | | |
| 10 | CPGP-5-3 | 3.5 | | | |
| $\Sigma$ | | 100.0 | | | |

Example 3 (Comparative)

A liquid-crystalline mixture M-3 having the composition and properties as indicated in the following table is prepared.

| Composition | | | Physical Properties | | |
|---|---|---|---|---|---|
| Compound No. | Abbreviation | Conc. / mass-% | T(N, I) | = 91 | °C |
| 1 | PZG-4-N | 6.0 | $\Delta\varepsilon$(20°C, 1 kHz) | = 3.6 | |
| 2 | PPTUI-3-2 | 10.0 | $\gamma_1$ (20°C) | = 231 | mPa · s |
| 3 | PPTUI-3-4 | 16.0 | tan $\delta_{\varepsilon r,\perp}$ (20°C, 19 GHz) | = 0.0138 | |
| 4 | PPTUI-4-4 | 30.0 | tan $\delta_{\varepsilon r,\parallel}$ (20°C, 19 GHz) | = 0.0049 | |
| 5 | PTP-3-5 | 16.0 | $\tau$ (20°C, 19 GHz) | = 0.211 | |
| 6 | PTP-4-5 | 16.0 | $\eta$ (20°C, 19 GHz) | = 15.3 | |
| 7 | CC-3-V | 6.0 | | | |
| $\Sigma$ | | 100.0 | | | |

Example 4

A liquid-crystalline mixture M-4 having the composition and properties as indicated in the following table is prepared.

| Composition | | | Physical Properties | | |
|---|---|---|---|---|---|
| Compound No. | Abbreviation | Conc. / mass-% | T(N, I) | = 98 | °C |
| 1 | PPTUI-3-2 | 10.0 | $\Delta n$(20°C,589.3 nm) | = 0.265 | |
| 2 | PPTUI-3-4 | 15.0 | $\Delta\varepsilon$(20°C, 1 kHz) | = 2.1 | |
| 3 | PPTUI-4-4 | 30.0 | $\gamma_1$ (20°C) | = 220 | mPa · s |
| 4 | PTP-3-5 | 14.0 | tan $\delta_{\varepsilon r,\perp}$ (20°C, 19 GHz) | = 0.0104 | |
| 5 | PTP-4-5 | 14.0 | tan $\delta_{\varepsilon r,\parallel}$ (20°C, 19 GHz) | = 0.0037 | |
| 6 | PGUQU-3-F | 2.0 | $\tau$ (20°C, 19 GHz) | = 0.209 | |
| 7 | PGUQU-5-F | 3.0 | $\eta$ (20°C, 19 GHz) | = 20.1 | |
| 8 | CC-3-V | 12.0 | | | |
| $\Sigma$ | | 100.0 | | | |

Example 5

A liquid-crystalline mixture M-5 having the composition and properties as indicated in the following table is prepared.

| Composition | | | Physical Properties | | |
|---|---|---|---|---|---|
| Compound No. | Abbreviation | Conc. / mass-% | T(N, I) | = 102 | °C |
| 1 | PPTUI-3-2 | 20.0 | $\Delta n$(20°C,589.3 nm) | = 0.257 | |
| 2 | PPTUI-3-4 | 30.0 | $\Delta\varepsilon$(20°C, 1 kHz) | = 3.5 | |
| 3 | PTP-3-5 | 10.0 | $\gamma_1$ (20°C) | = 172 | mPa · s |
| 4 | PTP-4-5 | 10.0 | tan $\delta_{\varepsilon r,\perp}$ (20°C, 19 GHz) | = 0.0110 | |
| 5 | PGUQU-3-F | 3.0 | tan $\delta_{\varepsilon r,\parallel}$ (20°C, 19 GHz) | = 0.0036 | |
| 6 | PGUQU-5-F | 7.0 | $\tau$ (20°C, 19 GHz) | = 0.208 | |
| 7 | CC-3-V | 20.0 | $\eta$ (20°C, 19 GHz) | = 19.0 | |
| $\Sigma$ | | 100.0 | | | |

Example 6 (Comparative)

A liquid-crystalline mixture M-6 having the composition and properties as indicated in the following table is prepared.

| Composition | | | Physical Properties | | |
|---|---|---|---|---|---|
| Compound No. | Abbreviation | Conc. / mass-% | T(N, I) | = 116 | °C |
| 1 | PZG-4-N | 10.0 | $\Delta n$(20°C,589.3 nm) | = 0.260 | |
| 2 | PPTUI-3-2 | 20.0 | $\Delta\varepsilon$(20°C, 1 kHz) | = 6.0 | |
| 3 | PPTUI-3-4 | 30.0 | $\gamma_1$ (20°C) | = 197 | mPa · s |
| 4 | PGP-2-2V | 10.0 | tan $\delta_{\varepsilon r,\perp}$ (20°C, 19 GHz) | = 0.0174 | |
| 5 | PGP-2-3 | 10.0 | tan $\delta_{\varepsilon r,\parallel}$ (20°C, 19 GHz) | = 0.0055 | |
| 6 | CC-3-V | 20.0 | $\tau$ (20°C, 19 GHz) | = 0.200 | |

(continued)

| Composition | | | Physical Properties | | |
|---|---|---|---|---|---|
| Compound | | Conc. | | | |
| Σ | | 100.0 | η (20°C, 19 GHz) | = 11.5 | |

Example 7 (Comparative)

A liquid-crystalline mixture M-7 having the composition and properties as indicated in the following table is prepared.

| Composition | | | Physical Properties | | |
|---|---|---|---|---|---|
| Compound No. | Abbreviation | Conc. / mass-% | T(N, I) | = 86 | °C |
| 1 | PZG-4-N | 7.0 | Δn(20°C,589.3 nm) | = 0.245 | |
| 2 | PPTUI-3-2 | 10.0 | Δε(20°C, 1 kHz) | = 3.8 | |
| 3 | PPTUI-3-4 | 15.0 | $\gamma_1$ (20°C) | = 163 | mPa · s |
| 4 | PPTUI-4-4 | 25.0 | tan $\delta_{\varepsilon r,\perp}$ (20°C, 19 GHz) | = 0.0146 | |
| 5 | PTP-3-5 | 13.0 | tan $\delta_{\varepsilon r,\parallel}$ (20°C, 19 GHz) | = 0.0050 | |
| 6 | PTP-4-5 | 13.0 | τ (20°C, 19 GHz) | = 0.195 | |
| 7 | CC-3-V | 17.0 | η (20°C, 19 GHz) | = 13.3 | |
| Σ | | 100.0 | | | |

Example 8 (Comparative)

A liquid-crystalline mixture M-8 having the composition and properties as indicated in the following table is prepared.

| Composition | | | Physical Properties | | |
|---|---|---|---|---|---|
| Compound No. | Abbreviation | Conc. / mass-% | T(N, I) | = 90 | °C |
| 1 | PZG-4-N | 10.0 | Δn(20°C,589.3 nm) | = 0.245 | |
| 2 | PPTUI-3-2 | 20.0 | Δε(20°C, 1 kHz) | = 5.5 | |
| 3 | PPTUI-3-4 | 30.0 | $\gamma_1$ (20°C) | = 154 | mPa · s |
| 4 | PTP-3-5 | 10.0 | tan $\delta_{\varepsilon r,\perp}$ (20°C, 19 GHz) | = 0.0165 | |
| 5 | PTP-4-5 | 10.0 | tan $\delta_{\varepsilon r,\parallel}$ (20°C, 19 GHz) | = 0.0058 | |
| 6 | CC-3-V | 20.0 | τ (20°C, 19 GHz) | = 0.193 | |
| Σ | | 100.0 | η (20°C, 19 GHz) | = 11.6 | |

Example 9 (Comparative)

A liquid-crystalline mixture M-9 having the composition and properties as indicated in the following table is prepared.

| Composition | | | Physical Properties | | |
|---|---|---|---|---|---|
| Compound No. | Abbreviation | Conc. / mass-% | T(N, I) | = 88 | °C |
| 1 | PZG-5-N | 10.0 | Δn(20°C,589.3 nm) | = 0.2386 | |
| 2 | PPTUI-3-2 | 10.0 | Δε(20°C, 1 kHz) | = 5.2 | |

(continued)

| Composition | | | Physical Properties | | |
|---|---|---|---|---|---|
| Compound | | Conc. | | | |
| 3 | PPTUI-3-4 | 15.0 | $\gamma_1$ (20°C) | = 156 | mPa · s |
| 4 | PPTUI-4-4 | 25.0 | tan $\delta_{\varepsilon r,\perp}$ (20°C, 19 GHz) | = 0.0160 | |
| 5 | PTP-3-5 | 10.0 | tan $\delta_{\varepsilon r,\parallel}$ (20°C, 19 GHz) | = 0.0054 | |
| 6 | PTP-4-5 | 10.0 | $\tau$ (20°C, 19 GHz) | = 0.187 | |
| 7 | CC-3-V | 20.0 | $\eta$ (20°C, 19 GHz) | = 11.7 | |
| $\Sigma$ | | 100.0 | | | |

Example 10 (Comparative)

A liquid-crystalline mixture M-10 having the composition and properties as indicated in the following table is prepared.

| Composition | | | Physical Properties | | |
|---|---|---|---|---|---|
| Compound No. | Abbreviation | Conc. / mass-% | T(N, I) | = 67 | °C |
| 1 | PZG-4-N | 10.0 | $\Delta$n(20°C,589.3 nm) | = 0.232 | |
| 2 | PPTUI-3-2 | 20.0 | $\Delta\varepsilon$(20°C, 1 kHz) | = 5.0 | |
| 3 | PPTUI-3-4 | 30.0 | $\gamma_1$ (20°C) | = 147 | mPa · s |
| 4 | PTP-3-5 | 10.0 | tan $\delta_{\varepsilon r,\perp}$ (20°C, 19 GHz) | = 0.0160 | |
| 5 | PTP-4-5 | 10.0 | tan $\delta_{\varepsilon r,\parallel}$ (20°C, 19 GHz) | = 0.0064 | |
| 6 | PCH-32 | 20.0 | $\tau$ (20°C, 19 GHz) | = 0.181 | |
| $\Sigma$ | | 100.0 | $\eta$ (20°C, 19 GHz) | = 11.3 | |

Example 11

A liquid-crystalline mixture M-11 having the composition and properties as indicated in the following table is prepared.

| Composition | | | Physical Properties | | |
|---|---|---|---|---|---|
| Compound No. | Abbreviation | Conc. / mass-% | T(N, I) | = 106 | °C |
| 1 | PPTUI-3-2 | 20.0 | $\Delta$n(20°C,589.3 nm) | = 0.267 | |
| 2 | PPTUI-3-4 | 30.0 | $\Delta\varepsilon$(20°C, 1 kHz) | = 4.0 | |
| 3 | PTP-3-5 | 11.0 | $\gamma_1$ (20°C) | = 196 | mPa · s |
| 4 | PTP-4-5 | 11.0 | | | |
| 5 | PGUQU-3-F | 3.0 | | | |
| 6 | PGUQU-5-F | 7.0 | | | |
| 7 | PPGU-3-F | 2.0 | | | |
| 8 | CC-3-V | 16.0 | | | |
| $\Sigma$ | | 100.0 | | | |

Example 12

A liquid-crystalline mixture M-12 having the composition and properties as indicated in the following table is prepared.

| Composition | | | Physical Properties | | |
|---|---|---|---|---|---|
| Compound No. | Abbreviation | Conc. / mass-% | T(N, I) | = 105 | °C |
| 1 | PPTUI-3-2 | 20.0 | Δn(20°C,589.3 nm) | = 0.267 | |
| 2 | PPTUI-3-4 | 30.0 | Δε(20°C, 1 kHz) | = 3.9 | |
| 3 | PTP-3-5 | 11.0 | $\gamma_1$ (20°C) | = 193 | mPa · s |
| 4 | PTP-4-5 | 11.0 | | | |
| 5 | PGUQU-3-F | 3.0 | | | |
| 6 | PGUQU-5-F | 7.0 | | | |
| 7 | PGIP-3-N | 2.0 | | | |
| 8 | CC-3-V | 16.0 | | | |
| Σ | | 100.0 | | | |

Example 13

A liquid-crystalline mixture M-13 having the composition and properties as indicated in the following table is prepared.

| Composition | | | Physical Properties | | |
|---|---|---|---|---|---|
| Compound No. | Abbreviation | Conc. / mass-% | T(N, I) | = 107 | °C |
| 1 | PPTUI-3-2 | 20.0 | Δn(20°C,589.3 nm) | = 0.266 | |
| 2 | PPTUI-3-4 | 28.0 | Δε(20°C, 1 kHz) | = 4.3 | |
| 3 | PTP-3-5 | 11.0 | $\gamma_1$ (20°C) | = 141 | mPa · s |
| 4 | PTP-4-5 | 11.0 | | | |
| 5 | PGUQU-4-F | 3.0 | | | |
| 6 | PGUQU-5-F | 7.0 | | | |
| 7 | PPGU-3-F | 2.0 | | | |
| 8 | PGIP-3-N | 2.0 | | | |
| 9 | CC-3-V | 16.0 | | | |
| Σ | | 100.0 | | | |

Example 14

A liquid-crystalline mixture M-14 having the composition and properties as indicated in the following table is prepared.

| Composition | | | Physical Properties | | |
|---|---|---|---|---|---|
| Compound No. | Abbreviation | Conc. / mass-% | T(N, I) | = 93 | °C |
| 1 | PP-1-2V1 | 9.0 | Δn(20°C,589.3 nm) | = 0.266 | |
| 2 | PPTUI-4-4 | 18.0 | Δε(20°C, 1 kHz) | = 3.0 | |
| 3 | PTP-2-O1 | 9.0 | $\gamma_1$ (20°C) | = 187 | mPa · s |
| 4 | PTP-3-O1 | 9.0 | | | |
| 5 | PTP-2-A1 | 4.5 | | | |
| 6 | PTP-3-A1 | 4.5 | | | |
| 7 | PGP-2-2V | 18.0 | | | |
| 8 | PGP-3-2V | 9.0 | | | |

(continued)

| Composition | | | Physical Properties | | |
|---|---|---|---|---|---|
| Compound | | Conc. | | | |
| 9 | PGUQU-5-F | 9.0 | | | |
| 10 | CC-3-V | 10.0 | | | |
| Σ | | 100.0 | | | |

Example 15

A liquid-crystalline mixture M-15 having the composition and properties as indicated in the following table is prepared.

| Composition | | | Physical Properties | | |
|---|---|---|---|---|---|
| Compound No. | Abbreviation | Conc. / mass-% | T(N, I) | = 101 | °C |
| 1 | PP-1-2V1 | 10.0 | Δn(20°C,589.3 nm) | = 0.297 | |
| 2 | PPTUI-4-4 | 20.0 | Δε(20°C, 1 kHz) | = 3.5 | |
| 3 | PTP-2-O1 | 10.0 | $\gamma_1$ (20°C) | = 273 | mPa . s |
| 4 | PTP-3-O1 | 10.0 | | | |
| 5 | PTP-2-A1 | 5.0 | | | |
| 6 | PTP-3-A1 | 5.0 | | | |
| 7 | PGP-2-2V | 20.0 | | | |
| 8 | PGP-3-2V | 10.0 | | | |
| 9 | PGUQU-5-F | 10.0 | | | |
| Σ | | 100.0 | | | |

Example 16

A liquid-crystalline mixture M-16 having the composition and properties as indicated in the following table is prepared.

| Composition | | | Physical Properties | | |
|---|---|---|---|---|---|
| Compound No. | Abbreviation | Conc. / mass-% | T(N, I) | = 105 | °C |
| 1 | CC-3-V | 20.0 | Δn(20°C,589.3 nm) | = 0.246 | |
| 2 | PPTUI-3-2 | 20.0 | Δε(20°C, 1 kHz) | = 5.0 | |
| 3 | PTP-2-O1 | 10.0 | $\gamma_1$ (20°C) | = 105 | mPa . s |
| 4 | PTP-3-O1 | 10.0 | | | |
| 5 | PGP-2-2V | 24.0 | | | |
| 6 | PGUQU-3-F | 3.0 | | | |
| 7 | PGUQU-4-F | 3.0 | | | |
| 8 | PGUQU-5-F | 5.0 | | | |
| 9 | PPGUQU-4-F | 5.0 | | | |
| Σ | | 100.0 | | | |

Example 17

A liquid-crystalline mixture M-17 having the composition and properties as indicated in the following table is prepared.

| Composition | | | Physical Properties | | |
|---|---|---|---|---|---|
| Compound No. | Abbreviation | Conc. / mass-% | T(N, I) | = 104 | °C |
| 1 | PPTUI-3-2 | 20.0 | Δn(20°C,589.3 nm) | = 0.269 | |
| 2 | PPTUI-3-4 | 28.0 | Δε(20°C, 1 kHz) | = 3.7 | |
| 3 | PTP-3-5 | 13.0 | $\gamma_1$ (20°C) | = 316 | mPa · s |
| 4 | PTP-4-5 | 13.0 | | | |
| 5 | PGUQU-5-F | 8.0 | | | |
| 6 | PPGU-3-F | 2.0 | | | |
| 7 | PGIP-3-N | 2.0 | | | |
| 8 | CC-3-V | 14.0 | | | |
| Σ | | 100.0 | | | |

Example 18

A liquid-crystalline mixture M-18 having the composition and properties as indicated in the following table is prepared.

| Composition | | | Physical Properties | | |
|---|---|---|---|---|---|
| Compound No. | Abbreviation | Conc. / mass-% | T(N, I) | = 102 | °C |
| 1 | PPTUI-3-2 | 20.0 | Δn(20°C,589.3 nm) | = 0.277 | |
| 2 | PPTUI-3-4 | 30.0 | | | |
| 3 | PTP-3-5 | 15.0 | | | |
| 4 | PTP-4-5 | 15.0 | | | |
| 5 | PGUQU-5-F | 8.0 | | | |
| 6 | PPGU-3-F | 2.0 | | | |
| 7 | CC-3-V | 10.0 | | | |
| Σ | | 100.0 | | | |

Example 19 (Comparative)

A liquid-crystalline mixture M-19 having the composition and properties as indicated in the following table is prepared.

| Composition | | | Physical Properties | | |
|---|---|---|---|---|---|
| Compound No. | Abbreviation | Conc. / mass-% | T(N, I) | = 106 | °C |
| 1 | PZG-4-N | 8.0 | Δn(20°C,589.3 nm) | = 0.261 | |
| 2 | PPTUI-3-2 | 20.0 | Δε(20°C, 1 kHz) | = 4.8 | |
| 3 | PPTUI-3-4 | 40.0 | $\gamma_1$ (20°C) | = 174 | mPa · s |
| 4 | PP-1-2V1 | 12.0 | | | |
| 5 | CC-3-V | 20.0 | | | |
| Σ | | 100.0 | | | |

Example 20 (Comparative)

A liquid-crystalline mixture M-20 having the composition and properties as indicated in the following table is

prepared.

| Composition | | | Physical Properties | | |
|---|---|---|---|---|---|
| Compound No. | Abbreviation | Conc. / mass-% | T(N, I) | = 94 | °C |
| 1 | PZG-4-N | 10.0 | Δn(20°C,589.3 nm) | = 0.253 | |
| 2 | PPTUI-3-2 | 20.0 | Δε(20°C, 1 kHz) | = 5.6 | |
| 3 | PPTUI-3-4 | 30.0 | $\gamma_1$ (20°C) | = 195 | mPa · s |
| 4 | PGP-2-2V | 10.0 | | | |
| 5 | PGP-2-3 | 10.0 | | | |
| 6 | PCH-32 | 20.0 | | | |
| Σ | | 100.0 | | | |

Example 21 (Comparative)

A liquid-crystalline mixture M-21 having the composition and properties as indicated in the following table is prepared.

| Composition | | | Physical Properties | | |
|---|---|---|---|---|---|
| Compound No. | Abbreviation | Conc. / mass-% | T(N, I) | = 129 | °C |
| 1 | PPTUI-3-2 | 20.0 | Δn(20°C,589.3 nm) | = 0.270 | |
| 2 | PPTUI-3-4 | 30.0 | Δε(20°C, 1 kHz) | = 3.7 | |
| 3 | PGP-2-2V | 10.0 | $\gamma_1$ (20°C) | = 220 | mPa . s |
| 4 | PGP-2-3 | 10.0 | | | |
| 5 | PGUQU-3-F | 3.0 | | | |
| 6 | PGUQU-5-F | 7.0 | | | |
| 7 | CC-3-V | 20.0 | | | |
| Σ | | 100.0 | | | |

Example 22 (Comparative)

A liquid-crystalline mixture M-22 having the composition and properties as indicated in the following table is prepared.

| Composition | | | Physical Properties | | |
|---|---|---|---|---|---|
| Compound No. | Abbreviation | Conc. / mass-% | T(N, I) | = 116 | °C |
| 1 | PPTUI-3-2 | 20.0 | Δn(20°C,589.3 nm) | = 0.267 | |
| 2 | PPTUI-3-4 | 38.0 | | | |
| 3 | PP-1-2V1 | 12.0 | | | |
| 4 | PGUQU-3-F | 3.0 | | | |
| 5 | PGUQU-5-F | 7.0 | | | |
| 6 | CC-3-V | 20.0 | | | |
| Σ | | 100.0 | | | |

Example 23

A liquid-crystalline mixture M-23 having the composition and properties as indicated in the following table is prepared.

| Composition | | | Physical Properties | | |
|---|---|---|---|---|---|
| Compound No. | Abbreviation | Conc. / mass-% | T(N, I) | = 101 | °C |
| 1 | PPTUI-3-2 | 20.0 | Δn(20°C,589.3 nm) | = 0.265 | |
| 2 | PPTUI-3-4 | 30.0 | Δε(20°C, 1 kHz) | = 3.5 | |
| 3 | PTP-3-5 | 12.0 | $\gamma_1$ (20°C) | = 185 | mPa . s |
| 4 | PTP-4-5 | 12.0 | | | |
| 5 | PGUQU-3-F | 3.0 | | | |
| 6 | PGUQU-5-F | 7.0 | | | |
| 7 | CC-3-V | 16.0 | | | |
| Σ | | 100.0 | | | |

Example 24 (Comparative)

A liquid-crystalline mixture M-24 having the composition and properties as indicated in the following table is prepared.

| Composition | | | Physical Properties | | |
|---|---|---|---|---|---|
| Compound No. | Abbreviation | Conc. / mass-% | T(N, I) | = 80 | °C |
| 1 | PZG-4-N | 10.0 | Δn(20°C,589.3 nm) | = 0.243 | |
| 2 | PPTUI-3-2 | 20.0 | Δε(20°C, 1 kHz) | = 5.3 | |
| 3 | PPTUI-3-4 | 30.0 | $\gamma_1$ (20°C) | = 168 | mPa · s |
| 4 | PGP-2-2V | 5.0 | | | |
| 5 | PGP-2-3 | 5.0 | | | |
| 6 | PTP-3-5 | 5.0 | | | |
| 7 | PTP-4-5 | 5.0 | | | |
| 8 | PCH-32 | 20.0 | | | |
| Σ | | 100.0 | | | |

Example 25 (Comparative)

A liquid-crystalline mixture M-25 having the composition and properties as indicated in the following table is prepared.

| Composition | | | Physical Properties | | |
|---|---|---|---|---|---|
| Compound No. | Abbreviation | Conc. / mass-% | T(N, I) | = 67 | °C |
| 1 | PZG-4-N | 8.50 | Δn(20°C,589.3 nm) | = 0.1516 | |
| 2 | CC-3-V | 50.00 | Δε(20°C, 1 kHz) | = 3.7 | |
| 3 | PPTUI-3-2 | 19.00 | $V_0$ (20°C) | = 1.87 | V |
| 4 | PTP-4-5 | 5.50 | $\gamma_1$ (20°C) | = 51 | mPa · s |
| 5 | PP-1-2V1 | 6.00 | $k_1$ (20°C) | = 11.6 | pN |
| 6 | PGP-2-2V | 11.00 | $k_3$ (20°C) | = 12.3 | pN |
| Σ | | 100.0 | tan $\delta_{\varepsilon,r,\perp}$ (20°C, 19 GHz) | = 0.0140 | |
| | | | tan $\delta_{\varepsilon r,\parallel}$ (20°C, 19 GHz) | = 0.0054 | |
| | | | τ (20°C, 19 GHz) | = 0.136 | |
| | | | η (20°C, 19 GHz) | = 9.6 | |

Example 26 (Comparative)

A liquid-crystalline mixture M-26 having the composition and properties as indicated in the following table is prepared.

| Composition | | | Physical Properties | | |
|---|---|---|---|---|---|
| Compound No. | Abbreviation | Conc. / mass-% | | | |
| | | | T(N, I) | = 173 | °C |
| 1 | PPTUI-3-2 | 20.0 | $\Delta n$(20°C,589.3 nm) | = 0.335 | |
| 2 | PPTUI-3-4 | 36.0 | $\Delta\varepsilon$(20°C, 1 kHz) | = 4.6 | |
| 3 | GGP-3-CL | 10.0 | $\gamma_1$ (20°C) | = 746 | mPa · s |
| 4 | GGP-5-CL | 20.0 | tan $\delta_{\varepsilon r,\perp}$ (20°C, 19 GHz) | = 0.0143 | |
| 5 | CPGP-5-2 | 7.0 | tan $\delta_{\varepsilon r,\parallel}$ (20°C, 19 GHz) | = 0.0038 | |
| 6 | CPGP-5-3 | 7.0 | $\tau$ (20°C, 19 GHz) | = 0.252 | |
| $\Sigma$ | | 100.0 | $\eta$ (20°C, 19 GHz) | = 17.6 | |

In comparison to the Examples 1 to 23, this mixture exhibits clearly a higher value for $\tau$.

## Claims

1. Liquid-crystalline medium for components, **characterized in that** it comprises at least one compound selected from the group of compounds of the formulae IB-1, IB-2 and IB-3

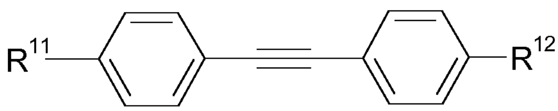

IB-1

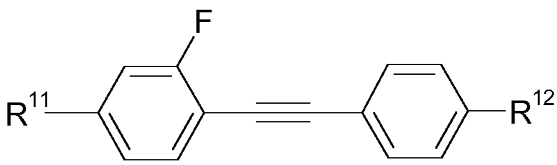

IB-2

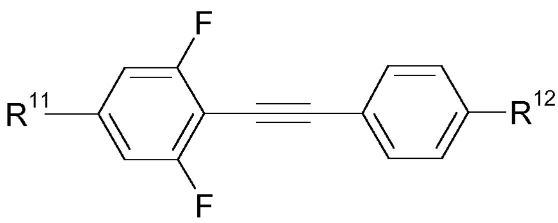

IB-3

wherein

$R^{11}$ denotes $C_nH_{2n+1}$
$R^{12}$ denotes $C_mH_{2m+1}$
m and n independently of one another, denote an integer in the range from 1 to 7,

and
one or more compounds of formula II

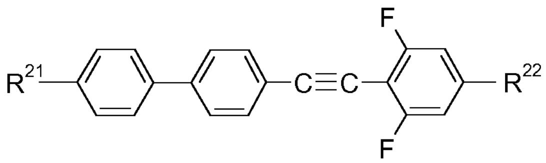

II

wherein

$R^{21}$ and $R^{22}$ are each independently F, Cl, CN, NCS or a straight-chain or branched alkyl group with 1 to 15 C atoms which may be unsubstituted, mono- or polysubstituted by halogen or CN, it being also possible for one or more non-adjacent CH2 groups to be replaced, in each occurrence independently from one another, by -O-, -S-, -NH-, $-N(CH_3)-$, -CO-, -COO-, -OCO-, -O-CO-O-, -S-CO-, -CO-S-, -CH=CH-, -CH=CF-, -CF=CF- or-C≡C- in such a manner that oxygen atoms are not linked directly to one another,

and
one or more compounds of formula III

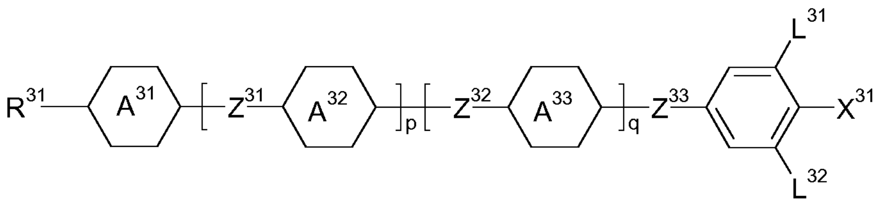

III

wherein

$R^{31}$ denotes F, Cl, CN, NCS or a straight-chain or branched alkyl group with 1 to 15 C atoms which may be unsubstituted, mono- or polysubstituted by halogen or CN, it being also possible for one or more non-adjacent CH2 groups to be replaced, in each occurrence independently from one another, by -O-, -S-, -NH-, $-N(CH_3)-$, -CO-, -COO-, -OCO-, -O-CO-O-, -S-CO-, -CO-S-, -CH=CH-, -CH=CF-, -CF=CF- or-C≡C- in such a manner that oxygen atoms are not linked directly to one another,

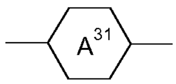 to 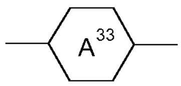

denote independently of one another, and in case $A^{32}$ and/or $A^{33}$ is/are present twice, also these independently of one another denote

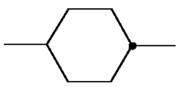, 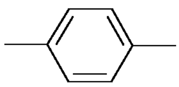, 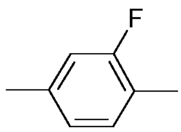,

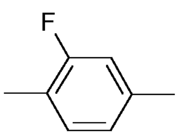, 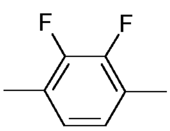 or 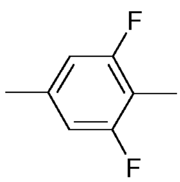,

wherein
$L^{31}$ and $L^{32}$ denote independently of one another H or F,
$X^{31}$ denotes halogen, halogenated alkyl or alkoxy with 1 to 3 C-atoms or halogenated alkenyl or alkenyloxy with

2 or 3 C-atoms or CN or NCS,
$Z^{31}$ to $Z^{32}$ denote independently of one another, and in case $Z^{31}$ and/or $Z^{32}$ is/are present twice, also these independently of one another denote $-CH_2CH_2-$, $-CF_2CF_2-$, -COO-, *trans*- -CH=CH-, *trans*- -CF=CF-, $-CH_2O-$, $-CF_2O-$ or a single bond,
$Z^{33}$ denotes $-CF_2O-$, and
p and q denote independently of one another 0, 1, 2 or 3, and wherein $p + q \geq 1$.

**2.** Liquid-crystalline medium according to Claim 1 , **characterized in that** it optionally comprises one or more compounds selected of formulae IV and V

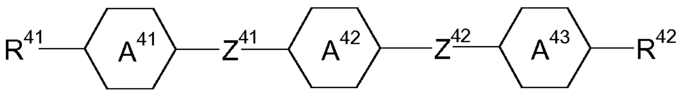

IV

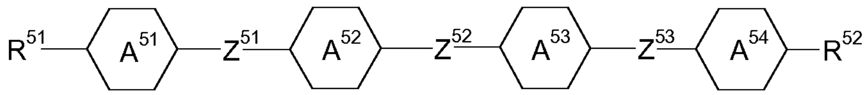

V

wherein

$R^{41}$ to $R^{52}$ independently of one another, denote unfluorinated alkyl or alkoxy having 1 to 15 or unfluorinated alkenyl, alkenyloxy or alkoxyalkyl having 2 to 15,
$Z^{41}$ and $Z^{53}$ independently of one another, denote $-CH_2CH_2-$, *trans*--CH=CH- or a single bond,

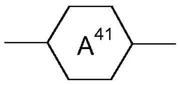

to

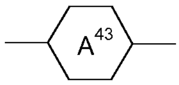

,

independently of one another, denote

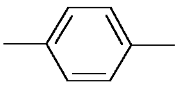

,

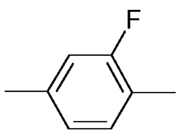

,

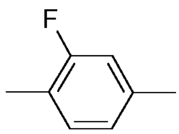

,

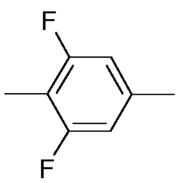

,

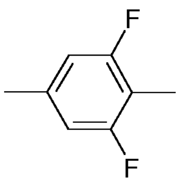

,

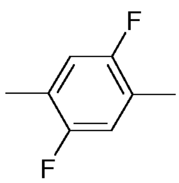

or

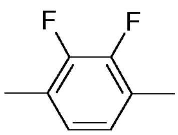

,

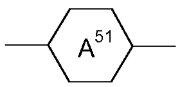

denotes

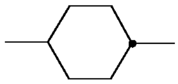,
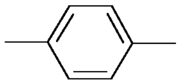,

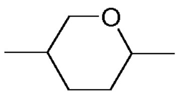 or 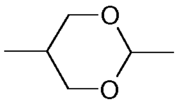,

and

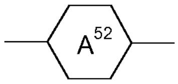 to 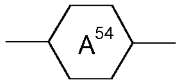

independently of one another, denote

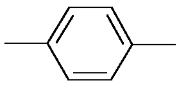, 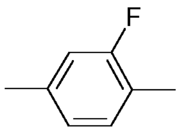, 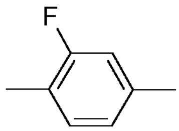,

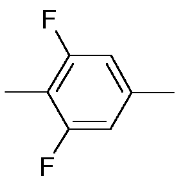, 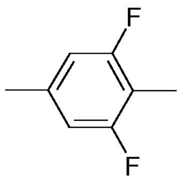, 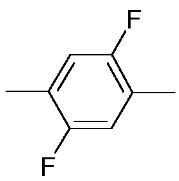

or

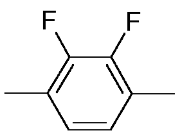.

**3.** Liquid-crystalline medium according to Claim 1 or 2, **characterized in that** it comprises one, two or more compounds of the formulae IA or IC

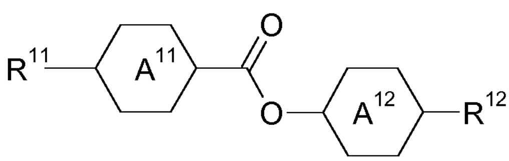

IA

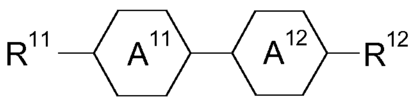

IC

wherein

$R^{11}$ and $R^{12}$ are each independently F, Cl, CN, NCS or a straight-chain or branched alkyl group with 1 to 15 C atoms which may be unsubstituted, mono- or polysubstituted by halogen or CN, it being also possible for one or more non-adjacent CH2 groups to be replaced, in each occurrence independently from one another, by -O-, -S-, -NH-, $-N(CH_3)-$, -CO-, -COO-, -OCO-, -O-CO-O-, -S-CO-, -CO-S-, -CH=CH-, -CH=CF-, -CF=CF- or-C≡C- in such a manner that oxygen atoms are not linked directly to one another, , and

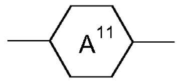
and
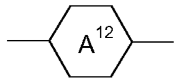

have the meanings indicated in Claim 1.

4. Liquid-crystalline medium according to one or more of Claims 1 to 3, **characterized in that** the proportion of compounds of the formula IB in the mixture as a whole is in the range from 5 to 60 % by weight.

5. Liquid-crystalline medium according to one or more of Claims 1 to 4, **characterized in that** the proportion of compounds of the formula II in the mixture as a whole is in the range from 10 to 80 % by weight.

6. Liquid-crystalline medium according to one or more of Claims 1 to 5, **characterized in that** the proportion of compounds of the formula III in the mixture as a whole is in the range from 1 to 30 % by weight.

7. Liquid-crystalline medium according to one or more of Claims 1 to 6, **characterized in that** the proportion of compounds of the formula IV and V in the mixture as a whole is in the range from 1 to 40 % by weight.

8. Process for the preparation of a liquid-crystalline medium according to one or more of Claims 1 to 7, **characterized in that** the compounds of the formulae IB, II and III are mixed with at least one further liquid-crystalline compound, and additives are optionally added.

9. Component for high-frequency technology, **characterized in that** it comprises a liquid-crystalline medium according to one or more of Claims 1 to 7.

10. Component according to Claim 10, **characterized in that** it is suitable for operation in the microwave range.

11. Component according to Claim 10 or 11, **characterized in that** it is a phase shifter.

12. Use of a liquid-crystal medium according to one or more of claims 1 to 7 in a component for high-frequency technology.

13. Microwave device, **characterized in that** it comprises one or more components according to one or more of Claims 10 to 12.

**Patentansprüche**

1. Flüssigkristallines Medium für Bauteile, **dadurch gekennzeichnet, dass** es mindestens eine Verbindung ausgewählt aus der Gruppe der Verbindungen der Formeln IB-1, IB-2 und IB-3

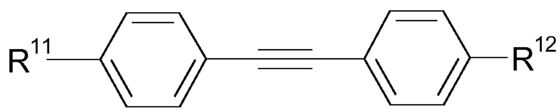
IB-1

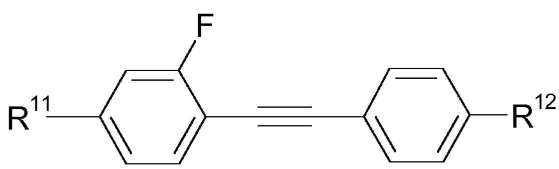
IB-2

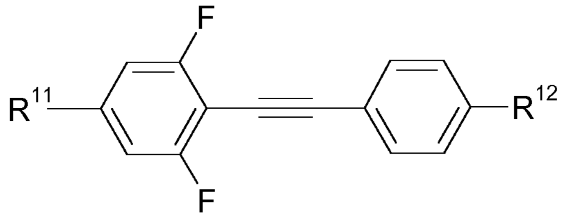

IB-3

bei denen

$R^{11}$ $C_nH_{2n+1}$ bedeutet,
$R^{12}$ $C_mH_{2m+1}$ bedeutet,
m und n unabhängig voneinander eine ganze Zahl im Bereich von 1 bis 7 bedeuten,

und
eine oder mehrere Verbindungen der Formel II

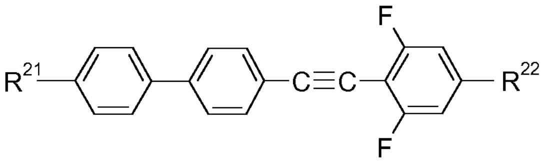

II

bei der

$R^{21}$ und $R^{22}$ jeweils unabhängig F, Cl, CN, NCS oder eine geradket-tige oder verzweigte Alkylgruppe mit 1 bis 15 C-Atomen sind, die unsubstituiert oder ein- oder mehrfach durch Halogen oder CN substituiert sein kann, wobei auch eine oder mehrere nicht benachbarte $CH_2$-Gruppen bei jedem Auftreten unabhängig voneinander so durch -O-, -S-, -NH-, -N($CH_3$)-, -CO-, -COO-, -OCO-, -O-CO-O-, -S-CO-, -CO-S-, -CH=CH-, -CH=CF-, -CF=CF- oder -C≡C- ersetzt sein können, dass Sauerstoffatome nicht direkt miteinander verknüpft sind, und eine oder mehrere Verbindungen der Formel III

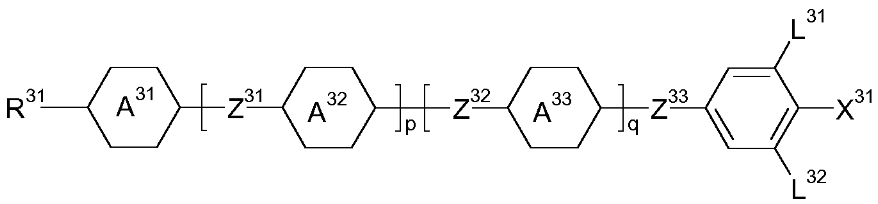

III

bei der

$R^{31}$ F, Cl, CN, NCS oder eine geradkettige oder verzweigte Alkylgruppe mit 1 bis 15 C-Atomen bedeutet, die un-substituiert oder ein- oder mehrfach durch Halogen o-der CN substituiert sein kann, wobei auch eine oder mehrere nicht benachbarte $CH_2$-Gruppen bei jedem Auftreten unabhängig voneinander so durch -O-, -S-, -NH-, -N($CH_3$)-, -CO-, -COO-, -OCO-, -O-CO-O-, -S-CO-, -CO-S-, -CH=CH-, -CH=CF-, -CF=CF- oder -C≡C-ersetzt sein können, dass Sauerstoffatome nicht direkt miteinander verknüpft sind,

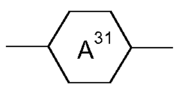

bis

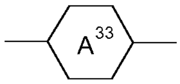

unabhängig voneinander und, wenn $A^{32}$ und/oder $A^{33}$ zweimal vorhanden ist/sind, auch diese unabhängig voneinander

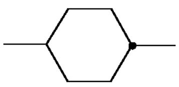, 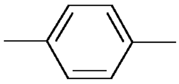, 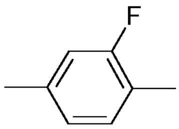,

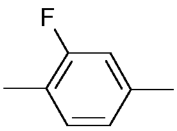, 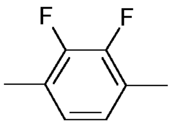 oder 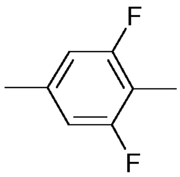

bedeuten,
$L^{31}$ und $L^{32}$ unabhängig voneinander H oder F bedeuten,
$X^{31}$ Halogen, halogeniertes Alkyl oder Alkoxy mit 1 bis 3 C-Atomen oder halogeniertes Alkenyl oder Alkenyloxy mit 2 oder 3 C-Atomen oder CN oder NCS bedeutet,
$Z^{31}$ bis $Z^{33}$ unabhängig voneinander und, wenn $Z^{31}$ und/oder $Z^{32}$ zweimal vorhanden ist/sind, auch diese unabhängig voneinander $-CH_2CH_2-$, $-CF_2CF_2-$, -COO-, *trans*-CH=CH-, trans-CF=CF-, $-CH_2O-$, $-CF_2O-$ oder eine Einfachbindung bedeuten,
$Z^{33}$ $-CF_2O-$ bedeutet und
p und q unabhängig voneinander 0, 1, 2 oder 3 bedeuten und bei denen $p + q \geq 1$,

enthält.

**2.** Flüssigkristallines Medium nach Anspruch 1, **dadurch gekennzeichnet, dass** es gegebenenfalls eine oder mehrere Verbindungen enthält, die aus den Formeln IV und V

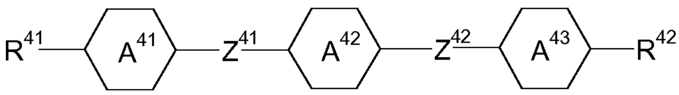

IV

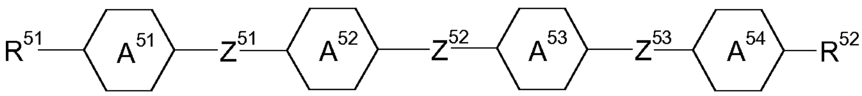

V

ausgewählt sind, bei denen

$R^{41}$ bis $R^{52}$ unabhängig voneinander unfluoriertes Alkyl oder Alkoxy mit 1 bis 15 C-Atomen oder unfluoriertes Alkenyl, Alkenyloxy oder Alkoxyalkyl mit 2 bis 15 C-Atomen bedeuten,
$Z^{41}$ und $Z^{53}$ unabhängig voneinander -CH2CH2-, trans-CH=CH- oder eine Einfachbindung bedeuten,

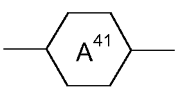

bis

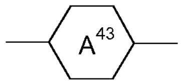

unabhängig voneinander

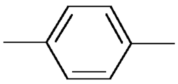 , 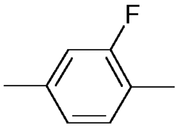 , 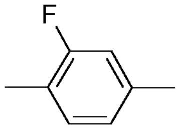 ,

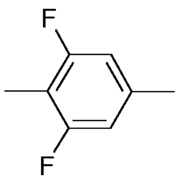 , 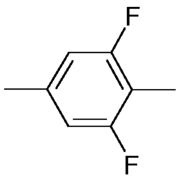 , 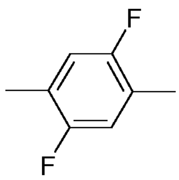

oder

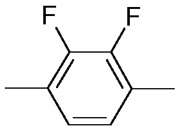

bedeuten,

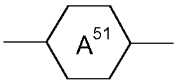

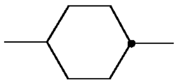 , 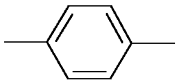 ,

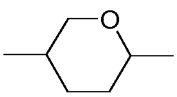 oder 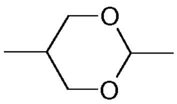

bedeutet und

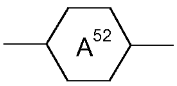

bis

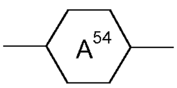

unabhängig voneinander

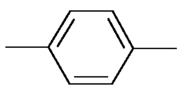 , 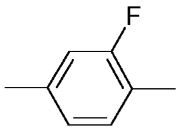 , 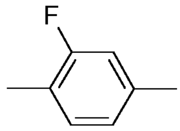 ,

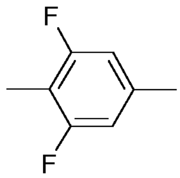 , 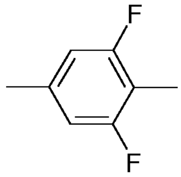 , 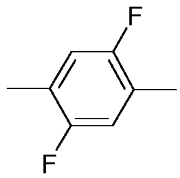

oder

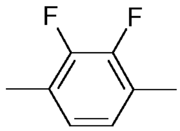

bedeuten.

3. Flüssigkristallines Medium nach Anspruch 1 oder 2, **dadurch gekennzeichnet, dass** es eine, zwei oder mehr Verbindungen der Formeln IA oder IC

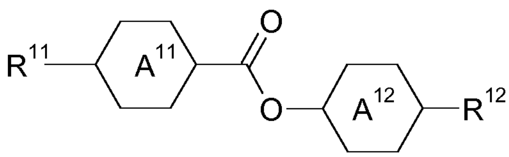

IA

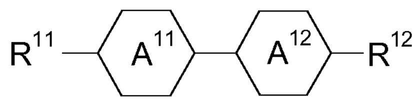

IC

enthält, bei denen

$R^{11}$ und $R^{12}$ jeweils unabhängig F, Cl, CN, NCS oder eine geradkettige oder verzweigte Alkylgruppe mit 1 bis 15 C-Atomen sind, die unsubstituiert oder ein- oder mehrfach durch Halogen oder CN substituiert sein kann, wobei auch eine oder mehrere nicht benachbarte $CH_2$-Gruppen bei jedem Auftreten unabhängig voneinander so durch -O-, -S-, -NH-, -N($CH_3$)-, -CO-, -COO-, -OCO-, -O-CO-O-, -S-CO-, -CO-S-, -CH=CH-, -CH=CF-, -CF=CF- oder -C≡C- ersetzt sein können, dass Sauerstoffatome nicht direkt miteinander verknüpft sind,

und

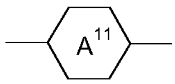 und 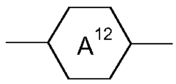

die in Anspruch 1 angegebenen Bedeutungen besitzen.

4. Flüssigkristallines Medium nach einem oder mehreren der Ansprüche 1 bis 3, **dadurch gekennzeichnet, dass** der Anteil der Verbindungen der Formel IB an der Gesamtmischung im Bereich von 5 bis 60 Gew.-% liegt.

5. Flüssigkristallines Medium nach einem oder mehreren der Ansprüche 1 bis 4, **dadurch gekennzeichnet, dass** der

Anteil der Verbindungen der Formel II an der Gesamtmischung im Bereich von 10 bis 80 Gew.-% liegt.

6. Flüssigkristallines Medium nach einem oder mehreren der Ansprüche 1 bis 5, **dadurch gekennzeichnet, dass** der Anteil der Verbindungen der Formel III an der Gesamtmischung im Bereich von 1 bis 30 Gew.-% liegt.

7. Flüssigkristallines Medium nach einem oder mehreren der Ansprüche 1 bis 6, **dadurch gekennzeichnet, dass** der Anteil der Verbindungen der Formel IV und V an der Gesamtmischung im Bereich von 1 bis 40 Gew.-% liegt.

8. Verfahren zur Herstellung eines flüssigkristallinen Mediums nach einem oder mehreren der Ansprüche 1 bis 7, **dadurch gekennzeichnet, dass** man die Verbindungen der Formeln IB, II und III mit mindestens einer weiteren flüssigkristallinen Verbindung mischt und gegebenenfalls Zusatzstoffe zugibt.

9. Bauteil für die Hochfrequenztechnik, **dadurch gekennzeichnet, dass** es ein flüssigkristallines Medium nach einem oder mehreren der Ansprüche 1 bis 7 enthält.

10. Bauteil nach Anspruch 9, **dadurch gekennzeichnet, dass** es sich für den Betrieb im Mikrowellenbereich eignet.

11. Bauteil nach Anspruch 9 oder 10, **dadurch gekennzeichnet, dass** es sich um einen Phasenschieber handelt.

12. Verwendung eines Flüssigkristallmediums nach einem oder mehreren der Ansprüche 1 bis 7 in einem Bauteil für die Hochfrequenztechnologie.

13. Mikrowellenvorrichtung, **dadurch gekennzeichnet, dass** sie ein oder mehrere Bauteile nach einem oder mehreren der Ansprüche 9 bis 11 enthält.

**Revendications**

1. Milieu cristallin liquide pour des composants, **caractérisé en ce qu'**il comprend au moins un composé qui est sélectionné parmi le groupe de composés des formules IB-1, IB-2 et IB-3 :

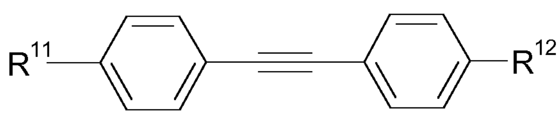

IB-1

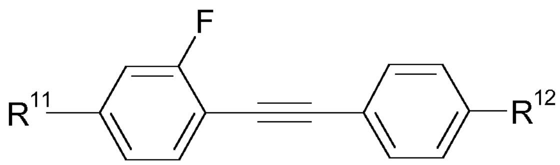

IB-2

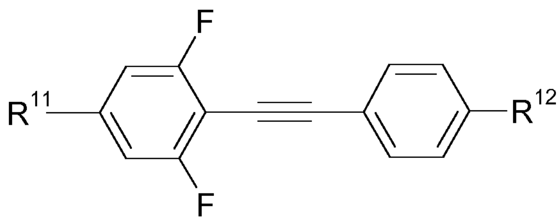

IB-3

dans lesquelles :

$R^{11}$ représente $C_nH_{2n+1}$ ;
$R^{12}$ représente $C_mH_{2m+1}$ ;
m et n représentent, de manière indépendante l'un de l'autre, un entier dans la plage de 1 à 7 ;

et

un ou plusieurs composé(s) de la formule II :

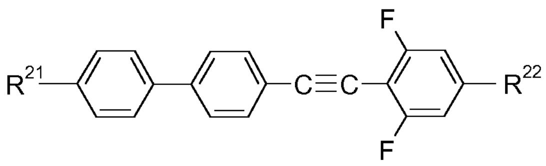

II

dans laquelle :

$R^{21}$ et $R^{22}$ sont chacun, de manière indépendante l'un de l'autre, F, Cl, CN, NCS ou un groupe alkyle en chaîne droite ou ramifié qui comporte de 1 à 15 atome(s) de C, lequel peut être non substitué, mono- ou polysubstitué par halogène ou par CN, étant entendu qu'il est également possible qu'un ou que plusieurs groupe(s) $CH_2$ non adjacents soi(en)t remplacé(s), pour chaque occurrence de manière indépendante les unes des autres, par -O-, -S-, -NH-, -N($CH_3$)-, -CO-, -COO-, -OCO-, -O-CO-O-, -S-CO-, -CO-S-, -CH=CH-, -CH=CF-, -CF=CF- ou-C≡C- de telle sorte que des atomes d'oxygène ne soient pas liés directement les uns aux autres ; et

et

un ou plusieurs composé(s) de la formule III :

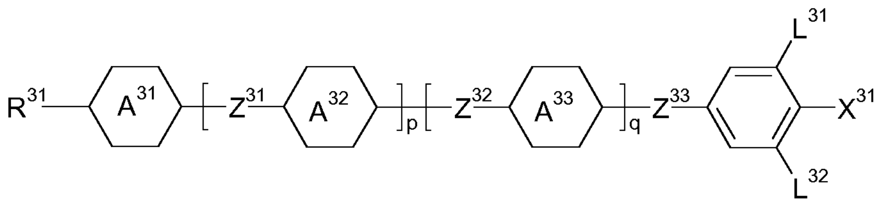

III

dans laquelle :

$R^{31}$ représente F, Cl, CN, NCS ou un groupe alkyle en chaîne droite ou ramifié qui comporte de 1 à 15 atome(s) de C, lequel peut être non substitué, mono- ou polysubstitué par halogène ou par CN, étant entendu qu'il est également possible qu'un ou que plusieurs groupe(s) CH2 non adjacents soi(en)t remplacé(s), pour chaque occurrence de manière indépendante les unes des autres, par -O-, -S-, -NH-, -N($CH_3$)-, -CO-, -COO-, -OCO-, -O-CO-O-, -S-CO-, -CO-S-, -CH=CH-, -CH=CF-, -CF=CF- ou -C≡C- de telle sorte que des atomes d'oxygène ne soient pas liés directement les uns aux autres ;

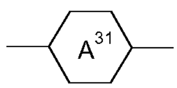 à 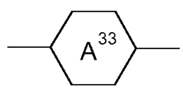

représentent, de manière indépendante les uns des autres, et dans le cas où $A^{32}$ et/ou $A^{33}$ sont/est présent(s) deux fois, ces occurrences représentent également, de manière indépendante l'une de l'autre :

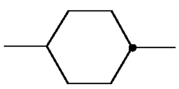, 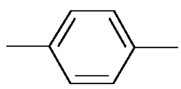, 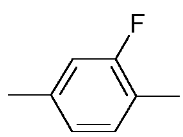,

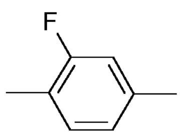, 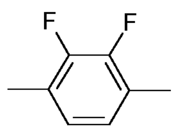 ou 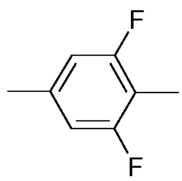,

$L^{31}$ et $L^{32}$ représentent, de manière indépendante l'un de l'autre, H ou F ;

$X^{31}$ représente halogène, alkyle ou alcoxy halogéné qui comporte de 1 à 3 atome(s) de C ou alkényle ou alkényloxy halogéné qui comporte 2 ou 3 atomes de C ou CN ou NCS;

$Z^{31}$ et $Z^{32}$ représentent, de manière indépendante l'un de l'autre, et dans le cas où $Z^{31}$ et/ou $Z^{32}$ sont/est présent(s) deux fois, ces occurrences représentent également, de manière indépendante l'une de l'autre, $-CH_2CH_2-$, $-CF_2CF_2-$, -COO-, *trans*- -CH=CH-, *trans*- -CF=CF-, $-CH_2O-$, $-CF_2O-$ ou une liaison simple ;

$Z^{33}$ représente $-CF_2O-$ ; et

p et q représentent, de manière indépendante l'un de l'autre, 0, 1, 2 ou 3, et où $p + q \geq 1$.

2. Milieu cristallin liquide selon la revendication 1, **caractérisé en ce qu'**il comprend de façon optionnelle un ou plusieurs composé(s) qui est/sont sélectionné(s) parmi les formules IV et V :

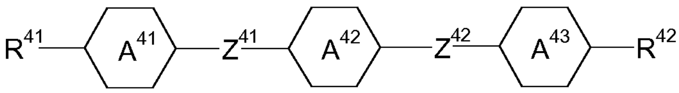

IV

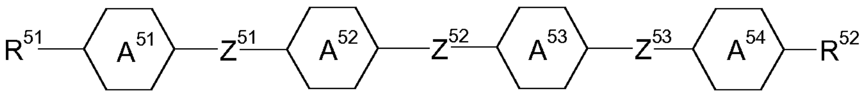

V

dans lesquelles :

$R^{41}$ à $R^{52}$ représentent, de manière indépendante les uns des autres, alkyle ou alcoxy non fluoré qui comporte de 1 à 15 atome(s) de C ou alkényle, alkényloxy ou alcoxy-alkyle non fluoré qui comporte de 2 à 15 atomes de C ;

$Z^{41}$ et $Z^{53}$ représentent, de manière indépendante l'un de l'autre, -CH2CH2-, *trans*- -CH=CH- ou une liaison simple ;

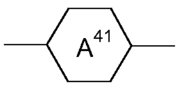

à

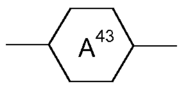

,

représentent, de manière indépendante les uns des autres :

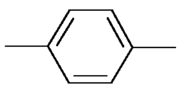

,

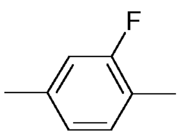

,

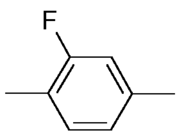

,

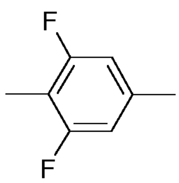

,

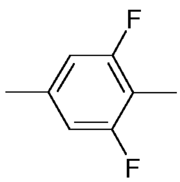

,

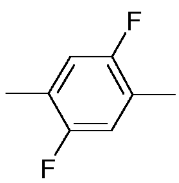

ou

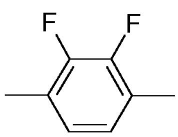

,

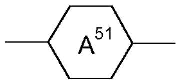

représente

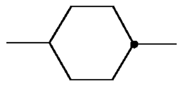 , 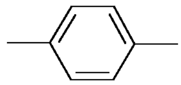 ,

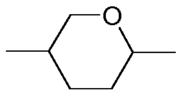 ou 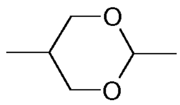 ;

et

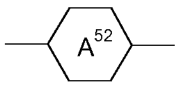 à 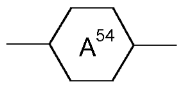

représentent, de manière indépendante les uns des autres :

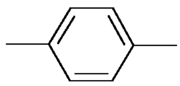 , 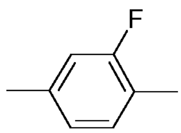 , 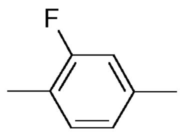 ,

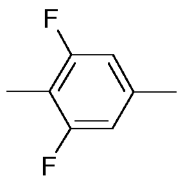 , 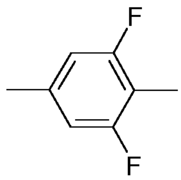 , 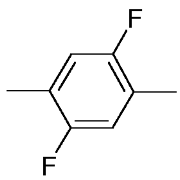

ou

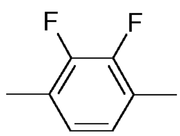 .

3. Milieu cristallin liquide selon la revendication 1 ou 2, **caractérisé en ce qu'**il comprend un composé, deux composés ou plus des formules IA ou IC :

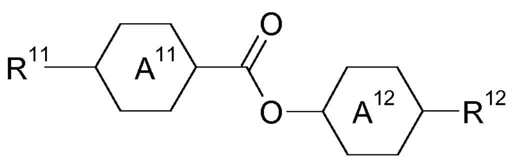

IA

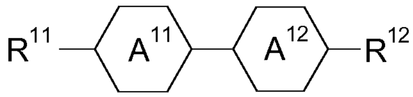

IC

dans lesquelles :

$R^{11}$ et $R^{12}$ sont, chacun de manière indépendante, F, Cl, CN, NCS ou un groupe alkyle en chaîne droite ou ramifié qui comporte de 1 à 15 atome(s) de C, lequel peut être non substitué, mono- ou polysubstitué par halogène ou par CN, étant entendu qu'il est également possible qu'un ou que plusieurs groupe(s) $CH_2$ non adjacents soi(en)t remplacé(s), pour chaque occurrence de manière indépendante les unes des autres, par -O-, -S-, -NH-, -N(CH3)-, -CO-, -COO-, -OCO-, -O-CO-O-, -S-CO-, -CO-S-, -CH=CH-, -CH=CF-, -CF=CF- ou -C≡C- de telle sorte que des atomes d'oxygène ne soient pas liés directement les uns aux autres ;

et

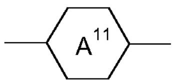

et

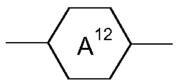

présentent les significations qui ont été indiquées selon la revendication 1.

4. Milieu cristallin liquide selon une ou plusieurs des revendications 1 à 3, **caractérisé en ce que** la proportion de composés de la formule IB dans le mélange pris dans sa globalité s'inscrit dans la plage qui va de 5 à 60 % en poids.

5. Milieu cristallin liquide selon une ou plusieurs des revendications 1 à 4, **caractérisé en ce que** la proportion de composés de la formule II dans le mélange pris dans sa globalité s'inscrit dans la plage qui va de 10 à 80 % en poids.

6. Milieu cristallin liquide selon une ou plusieurs des revendications 1 à 5, **caractérisé en ce que** la proportion de composés de la formule III dans le mélange pris dans sa globalité s'inscrit dans la plage qui va de 1 à 30 % en poids.

7. Milieu cristallin liquide selon une ou plusieurs des revendications 1 à 6, **caractérisé en ce que** la proportion de composés des formules IV et V dans le mélange pris dans sa globalité s'inscrit dans la plage qui va de 1 à 40 % en poids.

8. Procédé pour la préparation d'un milieu cristallin liquide selon une ou plusieurs des revendications 1 à 7, **caractérisé en ce que** les composés des formules IB, II et III sont mélangés avec au moins un autre composé cristallin liquide, et des additifs sont en option ajoutés.

9. Composant pour la technologie des hautes fréquences, **caractérisé en ce qu'**il comprend un milieu cristallin liquide selon une ou plusieurs des revendications 1 à 7.

10. Composant selon la revendication 9, **caractérisé en ce qu'**il est approprié pour un fonctionnement dans la plage des micro-ondes.

11. Composant selon la revendication 9 ou 10, **caractérisé en ce qu'**il s'agit d'un déphaseur.

12. Utilisation d'un milieu cristallin liquide selon une ou plusieurs des revendications 1 à 7 dans un composant pour la technologie des hautes fréquences.

13. Dispositif à micro-ondes, **caractérisé en ce qu'**il comprend un ou plusieurs composant(s) selon une ou plusieurs des revendications 9 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102010047404 A1
- DE 102010047702 A1
- DE 102010035987 A1
- WO 2011009524 A8
- DE 102004029429 A
- JP 2005120208 A
- DE 102010045370
- DE 102010051508
- WO 2011036243 A1

### Non-patent literature cited in the description


- **K.C. GUPTA ; R. GARG ; I. BAHL ; P. BHARTIA.** Microstrip Lines and Slotlines. Artech House, 1996
- **D. DOLFI ; M. LABEYRIE ; P. JOFFRE ; J.P. HUIGNARD.** Liquid Crystal Microwave Phase Shifter. *Electronics Letters,* May 1993, vol. 29 (10), 926-928
- **N. MARTIN ; N. TENTILLIER ; P. LAURENT ; B. SPLINGART ; F. HUERT ; PH. GELIN ; C. LEGRAND.** Electrically Microwave Tuneable Components Using Liquid Crystals. *32nd European Microwave Conference,* 2002, 393-396
- **WEIL, C.** Passiv steuerbare Mikrowellenphasenschieber auf der Basis nichtlinearer Dielektrika [Passively Controllable Microwave Phase Shifters based on Nonlinear Dielectrics. *Darmstädter Dissertationen,* 2002, vol. D17
- **C. WEIL ; G. LÜSSEM ; R. JAKOBY.** Tuneable Invert-Microstrip Phase Shifter Device Using Nematic Liquid Crystals. *IEEE MTT-S Int. Microw. Symp.,* June 2002, 367-370
- **T. KUKI ; H. FUJIKAKE ; H. KAMODA ; T. NOMOTO.** Microwave Variable Delay Line Using a Membrane Impregnated with Liquid Crystal. *IEEE MTT-S Int. Microwave Symp. Dig. 2002,* June 2002, 363-366
- **T. KUKI ; H. FUJIKAKE ; T. NOMOTO.** Microwave Variable Delay Line Using Dual-Frequency Switching-Mode Liquid Crystal. *IEEE Trans. Microwave Theory Tech.,* November 2002, vol. 50 (11), 2604-2609
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35GHz. *34th European Microwave Conference - Amsterdam,* 545-548
- Direct Simulation of Material Permittivites using an Eigen-Susceptibility Formulation of the Vector Variational Approach. **A. GAEBLER ; F. GOELDEN ; S. MÜLLER ; A. PENIRSCHKE ; R. JAKOBY.** 12MTC 2009 - International Instrumentation and Measurement Technology Conference. IEEE, 2009, 463-467
- Liquid Crystal Based Microwave Components with Fast Response Times: Materials, Technology, Power Handling Capability. **F. GOLDEN.** Dissertation. Technische Universität Darmstadt, 2009
- Single compounds and mixtures for microwave applications, Dielectric, microwave studies on selected systems. **A. LAPANIK.** Dissertation. Technische Universität Darmstadt, 2009
- **A. LAPANIK ; F. GOLDEN ; S. MÜLLER ; A. PENIRSCHKE ; R. JAKOBY ; W. HAASE.** *Frequenz,* 2011, vol. 65, 15-19
- **A. LAPANIK ; F. GOLDEN ; S. MÜLLER ; R. JAKOBY ; W. HAASE.** Highly birefringent nematic mixtures at room temperature for microwave applications. *Journal of Optical Engineering*
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35 GHz. *34th European Microwave Conference - Amsterdam,* 545-548
- Direct Simulation of Material Permittivites. **A. GAEBLER ; F. GOLDEN ; S. MÜLLER ; A. PENIRSCHKE ; R. JAKOBY.** 12MTC 2009 - International Instrumentation and Measurement Technology Conference. IEEE, 2009, 463-467
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35GHz. *34th European Microwave Conference,* 545-548
- *Pure Appl. Chem.,* 2001, vol. 73 (5), 888
- **C. TSCHIERSKE ; G. PELZL ; S. DIELE.** *Angew. Chem.,* 2004, vol. 116, 6340-6368

- Merck Liquid Crystals, Physical Properties of Liquid Crystals. Merck KGaA, November 1997